# EUROPEAN PATENT APPLICATION

(11) **EP 4 197 333 A1**
(43) Date of publication of application: **21.06.2023**
(21) Application number: 21214717.7
(22) Date of filing: 15.12.2021
(51) Int. Cl.: A01N 43/56, C07D 401/14

(54) **METHOD FOR CONTROLLING DIAMIDE RESISTANT PESTS & COMPOUNDS THEREFOR**

(71) Applicant: Syngenta Crop Protection AG, 4058 Basel (CH)
(72) Inventor: The designation of the inventor has not yet been filed
(74) Representative: SYNGENTA IP

(57) **Abstract**

A method for combating and controlling diamide-resistant insects to
(i) reduce damage on a plant, which comprises applying to the insect, to a locus of the insect, or to a plant susceptible to attack by the insect an, effective amount of a compound of formula I; or
(ii) protect plant propagation material, which comprises treating the propagation material or the site, where the propagation material is planted, with an effective amount of a compound of formula I;
wherein the compound of formula I is 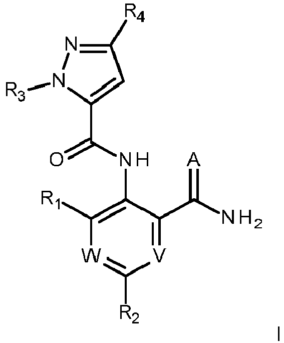 wherein the substituents are as defined in claim 1, and the agrochemically acceptable salts, stereoisomers, enantiomers, tautomers and N-oxides of those compounds, can be used as insecticides.

## Description

The present invention relates to method of controlling diamide-resistant pests by use of certain pesticidally active, in particular insecticidally active, diamide compounds. Further, present invention also relates to certain pesticidally active, in particular insecticidally active, diamide compounds, to processes for their preparation, to compositions comprising those compounds, and to their use for controlling animal pests, including arthropods and in particular insects or representatives of the order Lepidoptera, in particular diamide resistant Lepidoptera insects.

Heterocyclic diamide (or bisamide) derivatives with insecticidal action are known and described, for example, in WO2002070483, WO2004046129 and WO2006023783.

Bisamide insecticidal derivatives have been used widely during more than a decade and some insect populations have developed a level of resistance that renders them not susceptible enough to be sufficiently controlled by compounds of the bisamide class available on the market. The consequence of this evolution is that a higher dose of protectant must be used and / or the protection of the crops might be insufficient.

Diamide insecticides target the ryanodine receptor in insects and lead to a depletion of the intracellular calcium reservoirs (Ebbinghaus-Kintscher et al. 2006; Sattelle, Cordova, and Cheek 2008; Cordova et al. 2006). Commercial diamides can be attributed to two classes, the phthalic diamides with its sole representative being flubendiamide and the anthranilic diamides comprising chlorantraniliprole, cyantraniliprole, cyclaniliprole, and tetraniliprole. Other examples of phthalic diamides and anthranilic diamides are cyhalodiamide. fluchlordiniliprole and tetrachlorantraniliprole. All diamides share the same mode of action and so are grouped in the IRAC MoA Group 28.

The diamides represent a fast-growing class of insecticides introduced to the market since the commercialization of neonicotinoids (Sparks and Nauen 2015; Richardson et al. 2020; Troczka et al. 2017) and are extremely valuable insect control agents not least because they had exhibited little or no cross-resistance to older insecticide classes, which suffer markedly from resistance problems. However, reports of insect resistance to the diamides class of insecticides are on the increase.

The increase in resistance of such insects to diamide insecticides thus poses a significant threat to the cultivation of a number of commercially important crops, fruits and vegetables, and there is thus a need to find alternative insecticides capable of better controlling diamide resistant insects (e.g. to find insecticides that do not exhibit any cross-resistance with the diamide class).

Resistance may be defined as "a heritable change in the sensitivity of a pest population that is reflected in the repeated failure of a product to achieve the expected level of control when used according to the label recommendation for that pest species" (IRAC 2009).

Cross-resistance occurs when resistance to one insecticide confers resistance to another insecticide via the same biochemical mechanism. This can happen within insecticide chemical groups or between insecticide chemical groups. Cross-resistance may occur even if the resistant insect has never been exposed to one of the chemical classes of insecticide.

Two of the major mechanisms for diamide resistance include:
(i) Target site resistance, whereby resistance is associated with replacement of one or more amino acids in the insecticide target protein (i.e. the ryanodine receptor); and
(ii) Metabolic resistance, such as enhanced oxidative detoxification of diamides due to overexpression of cytochrome P450 monooxygenases (P450) or conjugation of diamides due to overexpression of UDP-dependent glycosyl transferases (UGT).

Target site resistance has been described in numerous Lepidopteran species incl. *Plutella xylostella* (Troczka et al. 2012; Steinbach et al. 2015; Guo et al. 2014), *Tuta absoluta* (Roditakis et al. 2017; Zimmer et al. 2019), *Spodoptera frugiperda* (Bolzan et al. 2019) *Spodoptera exigua* (Zuo et al. 2020, 2017), *Chilo suppressalis* (Yao et al. 2017; Yang et al. 2017). Similar to what has been described for target site resistance against other insecticides e.g. organochlorines affecting the GABA receptor (ffrench-Constant et al. 1998) parallel evolution can also be observed for diamide resistance with two mutations i.e. I4970M and G4946E (P. *xylostella* numbering) frequently described across species (Richardson et al. 2020). However, that does not exclude that mutations in different positions in the target-site may cause high levels of diamide resistance.

The cytochrome P450 monooxygenases are an important metabolic system involved in the detoxification of xenobiotics in phase I i.e. modification (Dermauw, Van Leeuwen, and Feyereisen 2020; Bard 2000). As such, P450 monooxygenases play an important role in insecticide resistance. P450 monooxygenases have such a phenomenal array of metabolizable substrates because of the presence of numerous P450s (~26-261) arthropodal species, as well as the broad substrate specificity of some P450s (Dermauw, Van Leeuwen, and Feyereisen 2020). Studies of monooxygenase-mediated resistance have indicated that resistance can be due to increased gene expression of one P450 involved (quantitative changes) in detoxification of the insecticide and might also be due to a mutation in the gene itself altering the amino acid composition (qualitative changes) (Feyereisen, Dermauw, and Van Leeuwen 2015). As such, metabolic cross-resistance mechanisms affect not only insecticides from the given class (e.g. neonicotinoids) but also seemingly unrelated insecticides. For example, cross-resistance relationships between the neonicotinoids and pymetrozine in *Bemisia tabaci* have been reported (Gorman et al. 2010; Nauen et al. 2013).

Apart from cytochrome P450s other enzyme and transport protein families may lead to insecticide resistance e.g. oxidases, hydrolases, transferases and ABC-transporters (Dermauw and Van Leeuwen 2014; Feyereisen, Dermauw, and Van Leeuwen 2015; Bass et al. 2014). P450s as well as other oxidases, transferases and ABC-transporters have been implicated in diamide resistance (Li et al. 2017; Mallott et al. 2019; Li et al. 2018; Shan et al. 2021).

Therefore, it is highly desirable to find classes of compounds offering a better control of the resistant insects.

It has now been surprisingly found that certain heterocyclic diamide derivatives are able to better control diamide-resistant insects, as well as other relevant insects.

The present invention accordingly relates, in a first aspect, to a method for combating and controlling diamide-resistant insects to
(i) reduce damage on a plant, which comprises applying to the insect, to a locus of the insect, or to a plant susceptible to attack by the insect, an effective amount of a compound of formula I; or
(ii) protect plant propagation material, which comprises treating the propagation material or the site, where the propagation material is planted, with an effective amount of a compound of formula I;
wherein the compound of formula I is 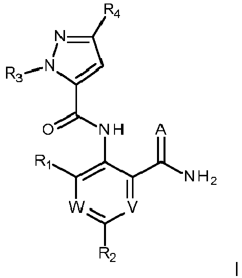 wherein
A is O or S;
V and W are independently CH or N, with the proviso that V and W cannot both be CH;
R₁ is hydrogen, halogen, C₁-C₆alkyl, C₁-C₆haloalkyl, or C₃-C₆cycloalkyl;
R₂ is hydrogen, halogen or cyano;
R₃ is phenyl, or a 6-membered heteroaromatic ring, each of which is unsubstituted or substituted with one to three substituents independently selected from R₆;
R₄ is hydrogen, halogen, C₁-C₆alkyl, C₁-C₆haloalkyl, C₃-C₆cycloalkyl, C₃-C₆halocycloalkyl, C₁-C₆alkoxy, C₁-C₆haloalkoxy, or CH₂-Y, where Y is C₃-C₆cycloalkyl, C₃-C₆halocycloalkyl, benzyloxy, halobenzyloxy, 5- or 6-membered heteroaromatic ring, which is unsubstituted or substituted with one to three groups independently selected from R₇, or a 9- or 10-membered heteroaromatic bicyclic system, which is unsubstituted or substituted with one to three groups independently selected from R₇; R₆ is independently selected from halogen, cyano, C₁-C₃alkyl, C₁-C₃haloalkyl, C₁-C₃alkoxy, and C₁-C₃haloalkoxy;
R₇ is independently selected from halogen, cyano, C₁-C₃alkyl, C₁-C₃haloalkyl, C₃-C₆cycloalkyl, phenyl, and phenyl substituted with one to three independently selected substituents from halogen, cyano, C₁-C₃alkyl, and C₁-C₃haloalkyl;
or an agronomically acceptable salt, isomer, enantiomer, tautomer and/or N-oxide of the compound of formula I.

It has further now been found that certain novel heterocyclic diamide derivatives provide improved control over these diamide-resistant insects. Accordingly, a second aspect of the present invention relates to a compound of formula I as defined in the first aspect with A, V, W, R₁, R₂, R₃, and R₄ as defined in the first aspect, and when
- R₃ is 3-chloro-2-pyridyl, V is N, W is CH, R₄ is bromo, chloro or CF₃, and R₁ is chloro, then R₂ is hydrogen, fluoro, bromo, iodo or cyano;
- R₃ is 3-chloro-2-pyridyl, V is N, W is CH, R₄ is bromo and R₁ is bromo, then R₂ is hydrogen, fluoro, chloro, iodo or cyano; and
- R₃ is 3-chloro-2-pyridyl, V is CH, W is N, R₄ is bromo and R₁ is chloro, then R₂ is hydrogen, fluoro, bromo, iodo or cyano.

In an embodiment of the first and second aspect, compounds having
(i) R₂ as chloro, R₃ as 3-chloro-2-pyridyl, V as N, W as CH, R₄ as bromo, chloro or CF₃, and R₁ as chloro;
(ii) R₂ as bromo, R₃ as 3-chloro-2-pyridyl, V as N, W as CH, R₄ as bromo and R₁ as bromo; and
(iii) R₂ as chloro, R₃ as 3-chloro-2-pyridyl, V as CH, W as N, R₄ as bromo and R₁ as chloro; are excluded.

Through the use of a compound of formula I according to each aspect of the present invention the damage caused on plants, especially on useful plants and ornamentals in agriculture, in horticulture and in forests, or on organs, such as fruits, flowers, foliage, stalks, tubers or roots, of such plants, and in some cases even plant organs which are formed at a later point in time, by a diamide-resistant insect is controlled, reduced.

Compounds I which have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine. Where appropriate, the corresponding internal salts can furthermore be formed. Preferred within the scope of the invention are agrochemically advantageous salts; however, the invention also encompasses salts which have disadvantage for agrochemical use, for example salts which are toxic to bees or fish, and which are employed, for example, for the isolation or purification of free compounds I or agrochemically utilizable salts thereof. Owing to the close relationship between the compounds I in free form and in the form of their salts, for the purposes of the invention the free compounds I or their salts hereinabove and hereinbelow are respectively to be understood as including, where appropriate, the corresponding salts or the free compounds I. The same applies analogously to tautomers of compounds I and salts thereof. In general, the free form is preferred in each case.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The compounds of formula I according to the invention also include hydrates which may be formed during the salt formation.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl.

The term "C₁-Cₙhaloalkyl" as used herein refers to a straight-chain or branched saturated alkyl radical attached via any of the carbon atoms having 1 to n carbon atoms (as mentioned above), where some or all of the hydrogen atoms in these radicals may be replaced by a halogen independently selected from fluorine, chlorine, bromine and iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3- pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. According a term "C₁-C₂fluoroalkyl" would refer to a C₁-C₂alkyl radical which carries 1, 2, 3, 4, or 5 fluorine atoms, for example, any one of difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl.

The term "C₁-Cₙalkoxy" as used herein refers to a straight-chain or branched saturated alkyl radical having 1 to n carbon atoms (as mentioned above) which is attached via an oxygen atom, i.e., for example, any one of the radicals methoxy, ethoxy, n-propoxy, 1-methylethoxy, n-butoxy, 1-methylpropoxy, 2-methylpropoxy or 1,1-dimethylethoxy.

The term "haloC₁-Cₙalkoxy" as used herein refers to a C₁-Cₙalkoxy radical where one or more hydrogen atoms on the alkyl radical is replaced by the same or different halo atom(s) - examples include tnfluoromethoxy, 2-fiuoroethoxy, 3-fluoropropoxy, 3,3,3-trifluoropropoxy, 4-chlorobutoxy.

The term "C₃-Cₙcycloalkyl" as used herein refers to 3-n membered cycloalkyl groups such as cyclopropane, cyclobutane, cyclopentane and cyclohexane.

The term "C₂-Cₙhalocycloalkyl" as used herein refers to a C₃-Cₙcycloalkyl moiety substituted with one or more halo atoms which may be the same or different.

Halogen or "halo" is generally fluorine, chlorine, bromine or iodine. This also applies, correspondingly, to halogen in combination with other meanings, such as haloalkyl

The term "6-membered heteroaromatic" refers to a 6 membered aromatic ring having 1 to 3 carbon atoms replaced independently by nitrogen, sulfur, or oxygen. Examples are pyridyl (or pyridinyl), pyridazinyl, pyrimidinyl, and pyrazinyl.

Examples of "5- or 6-membered heteroaromatic" refers to a 5 or 6 membered aromatic ring having 1 to 3 carbon atoms replaced independently by nitrogen, sulfur, or oxygen. Examples are pyridyl (or pyridinyl), pyridazinyl, pyrimidinyl, pyrazinyl, pyrrolyl, pyrazolyl, imidazolyl, triazolyl (e.g. 1.2.4 triazoyl), furanyl, thiophenyl, oxazolyl, isoxazolyl, oxadiazolyl, thiazolyl, isothiazolyl and thiadiazolyl.

Examples of "9- or 10-membered heteroaromatic" refers to a 9 or 10 membered aromatic ring made up of two rings, having 1 to 4 carbon atoms replaced independently by nitrogen, sulfur, or oxygen (the heteroatoms can be in one ring or distributed amongst the two). Examples are purinyl, quinolinyl, cinnolinyl, quinoxalinyl, indolyl, indazolyl, benzimidazolyl, benzothiophenyl, benzoxazolyl, benzothiazolyl, imidazo[1,2-a]pyridinyl, and imidazo[4,5-b]pyridinyl.

As used herein, the term "controlling" refers to reducing the number of pests (or insects), eliminating pests and/or preventing further pest damage such that damage to a plant or to a plant derived product is reduced. The insect encompasses all stages in the life cycle of the insect.

As used herein, the term "effective amount" refers to the amount of the compound, or a salt thereof, which, upon single or multiple applications provides the desired effect.

The staggered line as used herein, for example, in Ya1 to Ya17, represent the point of connection/ attachment to the rest of the compound.

An effective amount is readily determined by the skilled person in the art, by the use of known techniques and by observing results obtained under analogous circumstances. In determining the effective amount a number of factors are considered including, but not limited to: the type of plant or derived product to be applied; the pest to be controlled & its lifecycle; the particular compound applied; the type of application; and other relevant circumstances.

In a preferred embodiment of each aspect of the present invention, compound of formula I is represented by formula Ia 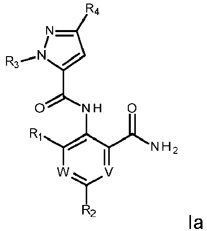 where V, W, R₁, R₂, R₃ and R₄ are as defined in the first aspect or second aspect.

In a further preferred embodiment of each aspect of the present invention, compound of formula I is represented by formula Ib, Ic or Id 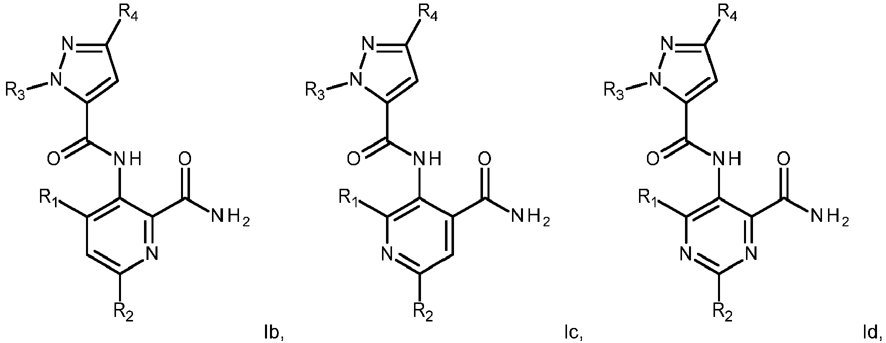 where R₁, R₂, R₃ and R₄ are as defined in the first aspect or second aspect.

Embodiments according to the present invention are provided as set out below.

In an embodiment of each aspect of the invention, A is
A. sulfur; or
B. oxygen.

In an embodiment of each aspect of the invention, W and V are
A. nitrogen and nitrogen respectively; or
B. nitrogen and carbon respectively; or
C. carbon and nitrogen respectively.

In an embodiment of each aspect of the invention, R₁ is
A. hydrogen, halogen, C₁-C₃alkyl, C₁-C₃haloalkyl, or C₃-C₄cycloalkyl; or
B. halogen, or C₁-C₃alkyl; or
C. methyl, F, Cl, Br, or I; or
D. methyl, Cl, or Br; or
E. methyl or chlorine.

In an embodiment of each aspect of the invention, R₂ is
A. halogen or cyano; or
B. chloro, bromo or cyano; or
C. chloro or bromo.

In an embodiment of each aspect of the invention, R₃ is
A. phenyl, or a pyridyl ring, each of which is substituted with one to three substituents independently selected from R₆; or
B. pyridyl ring, which is unsubstituted or is substituted with one to three substituents independently selected from R₆; or
C. pyridyl ring, which is substituted with one or two substituents independently selected from halogen, cyano, and C₁-C₃alkyl; or
D. pyridyl ring, which is substituted with one or two substituents independently selected from chlorine, bromine, iodine, cyano, and methyl; or
E. pyridyl ring, which is substituted with one or two substituents independently selected from chlorine, bromine, and iodine; or
F. pyridyl ring, which is substituted with one or two substituents chlorine; or
G. pyrid-2-yl ring, which is substituted with one or two substituents chlorine; or
H. 3-chloro-pyrid-2-yl ring or 3,5-dichloro-pyrid-2-yl ring.

In an embodiment of each aspect of the invention, R₄ is
A. hydrogen, halogen, C₁-C₃alkyl, C₁-C₃haloalkyl, C₃-C₄cycloalkyl, C₃-C₄halocycloalkyl, C₁-C₃alkoxy, C₁-C₃haloalkoxy, or CH₂-Y; or
B. halogen, C₁-C₃haloalkyl, C₁-C₃alkoxy, C₁-C₃haloalkoxy, or CH₂-Y; or
C. trifluoromethyl, bromine, chlorine, methoxy or CH₂-Y; or
D. trifluoromethyl; or
E. bromine; or
F. chlorine; or
G. methoxy; or
H. CH₂-Y.

In an embodiment of each aspect of the invention, Y is
A. C₃-C₄cycloalkyl, C₃-C₄halocycloalkyl, benzyloxy, halobenzyloxy, 5- or 6-membered heteroaromatic ring, which is unsubstituted or substituted with one to three groups independently selected from R₇, or a 9- or 10-membered heteroaromatic bicyclic system, which is unsubstituted or substituted with one to three groups independently selected from R₇; or
B. Any one of Ya to Yj; or 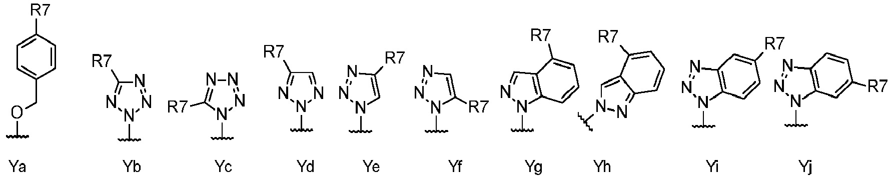
C. selected from Ya to Yh; or
D. Yb, Yc, Yd, Ye, Yf, Yg, or Yh; or
E. Yb, Yc, Yd, Ye or Yg, or Yh, where, independently of Y, R₇ is selected from chlorine, bromine, fluorine, difluoromethyl, trifluoromethyl, cyclopropyl or phenyl substituted by trifluoromethyl; or
F. selected from Ya1 to Ya17; or 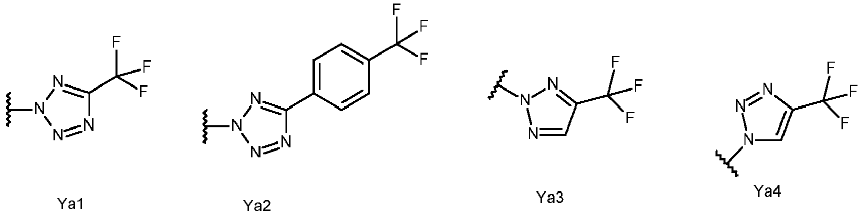 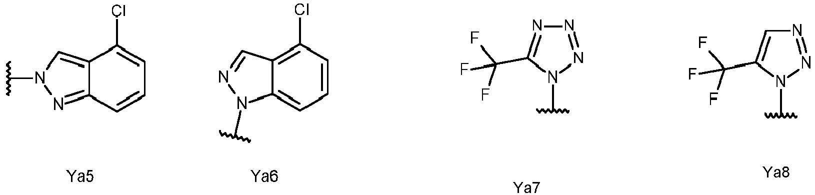 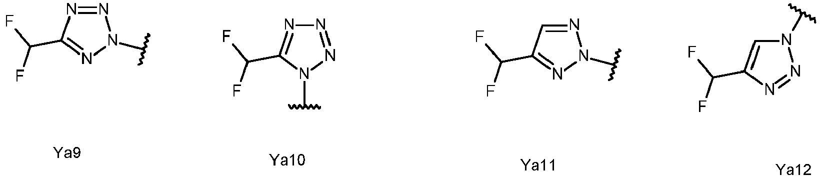 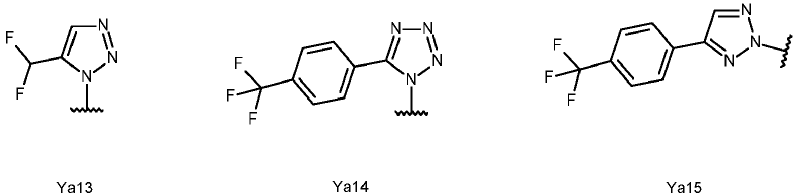 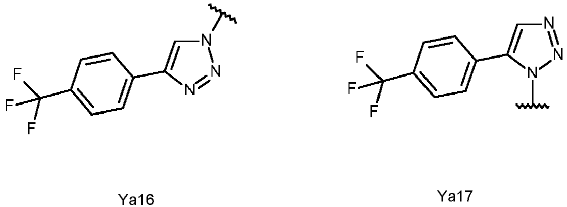
G. selected from Ya1 to Ya8; or
H. selected from Ya1 to Ya6.

In an embodiment of each aspect of the invention, R₆ is
A. independently selected from fluorine, chlorine, bromine, iodine, cyano, C₁-C₃alkyl, C₁-C₃haloalkyl, C₁-C₃alkoxy, and C₁-C₃haloalkoxy; or
B. independently selected from fluorine, chlorine, bromine, iodine, cyano, and C₁-C₃alkyl; or
C. independently selected from fluorine, chlorine, bromine, and iodine; or
D. chlorine.

In an embodiment of each aspect of the invention, R₇ is
A. independently selected from halogen, cyano, C₁-C₃alkyl, C₁-C₃haloalkyl, C₃-C₆cycloalkyl and phenyl substituted with one more selected from halogen, cyano, C₁-C₃alkyl, and C₁-C₃haloalkyl; or
B. independently selected from halogen, C₁-C₃alkyl, C₁-C₃haloalkyl, C₃-C₆cycloalkyl and phenyl substituted by chlorine, bromine, trifluoromethyl or difluoromethyl; or
C. independently selected from halogen, C₁-C₃haloalkyl, C₃-C₆cycloalkyl and phenyl substituted by chlorine, bromine, trifluoromethyl or difluoromethyl or
D. chlorine, bromine, fluorine, difluoromethyl, trifluoromethyl, cyclopropyl or phenyl substituted by trifluoromethyl.

In an embodiment of each aspect of the invention,
A. R₂ is not chloro when R₃ is 3-chloro-2-pyridyl, V is N, W is CH, R₄ is bromo, chloro or CF₃, and R₁ is chloro; or
B. R₂ is not bromo when R₃ is 3-chloro-2-pyridyl, V is N, W is CH, R₄ is bromo and R₁ is bromo; or
C. R₂ is not chloro when R₃ is 3-chloro-2-pyridyl, V is CH, W is N, R₄ is bromo and R₁ is chloro.

The present invention, accordingly, makes available a compound of formula I having the substituents A, W, V, R₁, R₂, R₃ and R₄, as defined above in all combinations / each permutation. Accordingly, made available, for example, is a compound of formula I with A being the first aspect (i.e. A is oxygen or sulfur); Wand V being embodiment C (i.e. carbon and nitrogen respectively); R₁ being embodiment D (i.e. methyl, Cl, or Br);); R₂ being embodiment A (i.e. halogen or cyano); R₃ being an embodiment E (i.e. pyridyl ring, which is substituted with one or two substituents independently selected from chlorine, bromine, and iodine); R₄ being embodiment C (i.e. trifluoromethyl, bromine, chlorine, methoxy or CH₂-Y); and Y is embodiment A (i.e. Y is C₃-C₄cycloalkyl, C₃-C₄halocycloalkyl, benzyloxy, halobenzyloxy, 5- or 6-membered heteroaromatic ring, which is unsubstituted or substituted with one to three groups independently selected from R₇, or a 9- or 10-membered heteroaromatic bicyclic system, which is unsubstituted or substituted with one to three groups independently selected from R₇, where R₇ is of embodiment B (i.e. R₇ is halogen, C₁-C₃alkyl, C₁-C₃haloalkyl, C₃-C₆cycloalkyl and phenyl substituted by chlorine, bromine, trifluoromethyl and difluoromethyl)).

In an embodiment of each aspect of the invention, the insects are those resistant to insecticides of the IRAC class 28 (https://irac-online.org > moa-classification), which act on the ryanodine receptors of the insects - such insecticides are generally referred to as diamides or phthalimide insecticides.

In an embodiment, the compounds of formula I control insects that demonstrate resistance to least one compound selected from chlorantraniliprole, cyantraniliprole, cyclantraniliprole, fluchlordiniliprole, tetrachlorantraniliprole, tetraniliprole, flubendiamide and cyhalodiamide.

The insects have developed target site resistance and have, for example, at least one of the mutations i.e. I4970M and G4946E (P. xylostella numbering). A skilled person would however not exclude that mutations in different positions in the target-site may also cause high levels of diamide resistance.

The diamide-resistant insects are preferably from the order Lepidoptera.

Preferred species are Plutella xylostella (Troczka et al. 2012; Steinbach et al. 2015; Guo et al. 2014), Tuta absoluta (Roditakis et al. 2017; Zimmer et al. 2019), Spodoptera frugiperda (Bolzan et al. 2019) Spodoptera exigua (Zuo et al. 2020, 2017), Chilo suppressalis (Yao et al. 2017; Yang et al. 2017).

In an embodiment of the first aspect, the method for combating and controlling diamide-resistant insect is in a defined area /field of plants where the ratio of diamide-resistant insects to their corresponding sensitive strains is greater than 1:20 (based on number of insects), preferably greater than 1:10, especially greater than 1:5.

In an embodiment of the first aspect, a compound of formula I controls the diamide-resistant insect better compared to the secondary amide analog of the compound of formula I. The improvement in control can be more than 20, preferably more than 30, more preferably more than 40, and most preferably more than 50, percent. The improvement in the control is assessed at the same level, for example at 5 ppm.

In an embodiment of the first aspect, the method for combating and controlling diamide-resistant insect is by applying to a plant susceptible to attack by the insect an effective amount of a compound of formula I; or by treating the propagation material with an effective amount of a compound of formula I.

In an embodiment, the compounds of formula I are represented by compounds of formulae la, Ib, Ic, and Id, where the corresponding substituents are as defined for formula I in each aspect and their embodiments.

In an embodiment of the first aspect of the invention, the compound of formula Ia has as W and V independently CH or N, with the proviso that V and W cannot both be CH; R₁ halogen, or C₁-C₃alkyl; R₂ as halogen or cyano; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl).

In an embodiment of the first aspect of the invention, the compound of formula Ia has as W and V independently CH or N, with the proviso that V and W cannot both be CH; R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl).

In an embodiment of the first aspect of the invention, the compound of formula Ia has as W and V independently CH or N, with the proviso that V and W cannot both be CH; R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya1 to Ya6).

In an embodiment of the first aspect of the invention, the compound of formula Ia has as W and V independently CH or N, with the proviso that V and W cannot both be CH; R₁ halogen, or C₁-C₃alkyl; R₂ as halogen; R₃ as 3-chloro-2-pyridyl; R₄ as halogen, C₁-C₃haloalkyl, C₁-C₃alkoxy, C₁-C₃haloalkoxy, or CH₂-Y (where Y is selected from Ya1 to Ya6).

In an embodiment of the first aspect of the invention, the compound of formula Ib, Ic or Id has as R₁ halogen, or C₁-C₃alkyl; R₂ as halogen or cyano; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl).

In an embodiment of the first aspect of the invention, the compound of formula Ib, Ic or Id has as R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl).

In an embodiment of the first aspect of the invention, the compound of formula Ib, Ic or Id has as R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya1 to Ya6).

In an embodiment of the first aspect of the invention, the compound of formula Ib, Ic or Id has as R₁ halogen, or C₁-C₃alkyl; R₂ as halogen; R₃ as 3-chloro-2-pyridyl; R₄ as halogen, C₁-C₃haloalkyl, C₁-C₃alkoxy, C₁-C₃haloalkoxy, or CH₂-Y (where Y is selected from Ya1 to Ya6).

In an embodiment of the first aspect of the invention, the compound of formula Ib, Ic or Id has as R₁ chlorine, bromine, or methyl; R₂ as chlorine or bromine; R₃ as 3-chloro-2-pyridyl; R₄ as bromine, trifluoromethyl, methoxy, difluoromethoxy, 1,1,1-trifluoroethoxy, or CH₂-Y (where Y is selected from Ya1 to Ya6).

In an embodiment of the first aspect of the invention, the compound of formula Ib has as R₁ halogen, or C₁-C₃alkyl; R₂ as halogen; R₃ as 3-chloro-2-pyridyl; R₄ as halogen, C₁-C₃haloalkyl, C₁-C₃alkoxy, C₁-C₃haloalkoxy, or CH₂-Y (where Y is selected from Ya1 to Ya6).

In an embodiment of the first aspect of the invention, the compound of formula Ib has as R₁ chlorine, bromine, or methyl; R₂ as chlorine or bromine; R₃ as 3-chloro-2-pyridyl; R₄ as bromine, trifluoromethyl, methoxy, difluoromethoxy, 1,1,1-trifluoroethoxy, or CH₂-Y (where Y is selected from Ya1 to Ya6).

Preferred compounds of formula I for the first aspect are those in Tables A-1 to A-7, B-1- to B-7, C-1 to C-7 and P and having the following CAS numbers and names: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide (hereinafter referred to as List Q).

Certain compounds of formula I are disclosed in WO2002070483, WO2004046129 and WO2006023783.

In an embodiment of each aspect of the invention, the compound of formula Ib, Ic, or Id has as R₁ halogen or C₁-C₃alkyl; R₂ as halogen or cyano, R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib, Ic or Id has as R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya to Yj, and R₇ is chlorine, bromine, fluorine, difluoromethyl, trifluoromethyl, cyclopropyl or phenyl substituted by trifluoromethyl), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib, Ic or Id has as R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya1 to Ya6), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib, Ic or Id has as R₁ halogen, or C₁-C₃alkyl; R₂ as halogen; R₃ as 3-chloro-2-pyridyl or 3,5-dichloro-2-pyridyl; R₄ as halogen, C₁-C₃haloalkyl, C₁-C₃alkoxy, C₁-C₃haloalkoxy, or CH₂-Y (where Y is selected from Ya1 to Ya6), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib, Ic or Id has as R₁ chlorine, bromine, or methyl; R₂ as chlorine or bromine; R₃ as 3-chloro-2-pyridyl; R₄ as bromine, trifluoromethyl, methoxy, difluoromethoxy, 1,1,1-trifluoroethoxy, or CH₂-Y (where Y is selected from Ya1 to Ya6), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib has as R₁ halogen, or C₁-C₃alkyl; R₂ as halogen; R₃ as 3-chloro-2-pyridyl; R₄ as halogen, C₁-C₃haloalkyl, C₁-C₃alkoxy, C₁-C₃haloalkoxy, or CH₂-Y (where Y is selected from Ya1 to Ya6), provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib has as R₁ chlorine, bromine, or methyl; R₂ as chlorine or bromine; R₃ as 3-chloro-2-pyridyl; R₄ as bromine, trifluoromethyl, methoxy, difluoromethoxy, 1,1,1-trifluoroethoxy, or CH₂-Y (where Y is selected from Ya1 to Ya6). provided the following CAS numbers and names are excluded: 458543-44-7, 458543-39-0, 458543-06-1, 4,6-dichloro-3-[[5-chloro-2-(3-chloro-2-pyridyl)pyrazole-3-carbonyl]amino]pyridine-2-carboxamide and 4,6-dichloro-3-[[2-(3-chloro-2-pyridyl)-5-(trifluoromethyl)pyrazole-3-carbonyl]amino]pyridine-2-carboxamide.

In an embodiment of each aspect of the invention, the compound of formula Ib, Ic, or Id has as R₁ halogen, or C₁-C₃alkyl; R₂ as halogen or cyano; R₃ as 3-chloro-2-pyridyl or 3,5-dichloro-2-pyridyl; R₄ as C₁-C₃alkoxy, C₁-C₃haloalkoxy, or CH₂-Y (where Y is selected from Ya1 to Ya6).

In an embodiment of each aspect of the invention, the compound of formula Ib, Ic, or Id has as R₁ methyl, chlorine or bromine; R₂ as bromine or chlorine; R₃ as 3-chloro-2-pyridyl or 3,5-dichloro-2-pyridyl; R₄ as methoxy, 1,1,1-trifluoroethoxy, difluoromethoxy or CH₂-Y (where Y is selected from Ya1 to Ya6).

In an embodiment of each aspect of the invention, the compound of formula Ib, Ic, or Id has as R₁ methyl, chlorine or bromine; R₂ as bromine or chlorine; R₃ as 3-chloro-2-pyridyl; R₄ as methoxy, 1,1,1-trifluoroethoxy, difluoromethoxy or CH₂-Y (where Y is selected from Ya1 to Ya6).

In a third aspect, the present invention makes available a composition comprising a compound of formula I as defined in the second aspect, one or more auxiliaries and diluent, and optionally one or more other active ingredient.

In a fourth aspect, the present invention makes available a method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound as defined in the second aspect or a composition as defined in the third aspect.

In a fifth aspect, the present invention makes available a method for the protection of plant propagation material from the attack by insects, acarines, nematodes or molluscs, which comprises treating the propagation material or the site, where the propagation material is planted, with an effective amount of a compound of formula I as defined in the second aspect or a composition as defined in the third aspect.

In a sixth aspect, the present invention makes available a plant propagation material, such as a seed, comprising, or treated with or adhered thereto, a compound of formula I as defined in the second aspect or a composition as defined in the third aspect.

Compounds of formula I can be prepared by those skilled in the art following known methods. More specifically compounds of formula I, and intermediates therefor can be prepared as described below in the schemes and examples. Certain stereogenic centers have been left unspecified for the clarity and are not intended to limit the teaching of the schemes in any way.

The process according to the invention for preparing compounds of formula I is carried out by methods known to those skilled in the art. In particular, WO 2002070483, WO 2004046129 and WO2006023783 describe methods for preparation of certain heterocyclic diamide compounds.

Compounds of formula I (when A is S) can be made, for example, from compounds of formula la, as depicted in reaction scheme 1, by treatment with a thionation reagent, as for example Lawesson's reagent or phosphorus sulfide (P₂S₅). The thionation of amides is well known and many examples are found in the literature. The compounds of formula I may have to be separated from regioisomers in case the regioselectivity of the thionation is not sufficient.

### Reaction Scheme 1: Preparation of Compounds of Formula I:

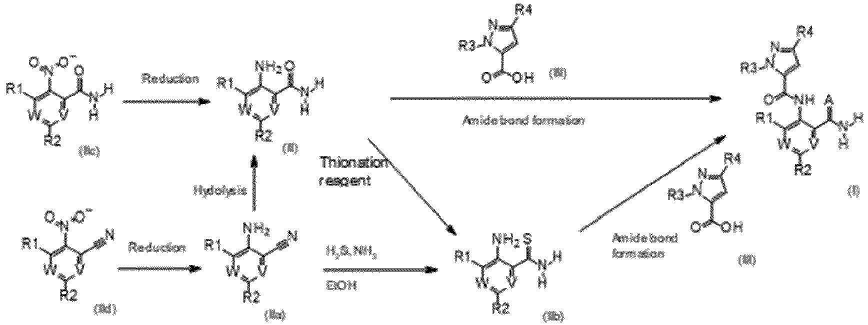

Compounds of formula I, wherein R₁, R₂, R₃, R₄, V, W and A are as defined in formula I can be obtained by condensation of a carboxylic acid of formula III, wherein R₃ and R₄ are as defined in formula I and an amino-amide compound of formula II, or an amino-thioamide of formula IIb, wherein R₁, R₂, V and Ware as defined in formula I. The amide bond formation is a very common reaction that can be performed under a large variety of conditions and with a variety of reagents and is well documented in the literature. The thioamides of formula IIb can be generated from the amides of formula II by using a thionation reagent like the Lawesson reagent or a phosphorus sulfide (P₄S₁₀). The reaction can be performed in toluene of an ether solvent, generally at elevated temperature. Another way of preparing amino-thioamides of formula IIb is the treat an aminonitrile compound of formula IIa with NH₃ and H₂S, for example in ethanol (as described by Alfred Taurins et al; Can. J. Chem. (1973), 51, 1741-1748). Hydrolysis of the aminonitriles of formula IIa can also be used to prepare the amino-amides of formula II. The amino derivatives of formulae II and IIa can be obtained from the reduction of the corresponding nitro derivatives IIc and IId. There is large choice of conditions for performing this operation, like Zn and NH₄Cl or SnCl₂.

There are many compounds of formulae II, IIa, IIc and IId that are commercially available or described in the literature. Other analogs can be prepared by analogy to those compounds. There are also many compounds of formula III that are described in the literature, including in a rich collection of patents, and other analogs can be prepared in a similar way by a person skilled in the art.

### Reaction Scheme 2: Preparation of Compounds of Formula I-a:

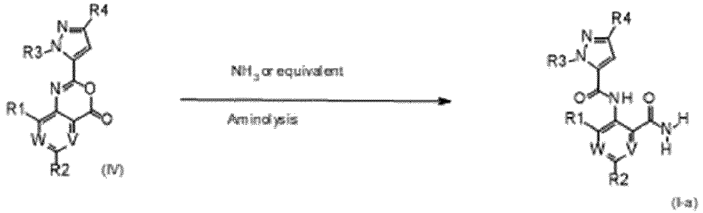

Compounds of formula I-a can also be prepared by aminolysis of fused oxazinones of formula IV, wherein R₁, R₂, R₃, R₄, V and W are as defined in formula I, by reacting them with ammonia or an equivalent, like for example ammonium acetate, or ammonium carbonate, in a solvent like, for example, ethyl acetate. The reaction can be performed at ambient temperature or, more conveniently at higher temperature, for example, up to the boiling temperature of the solvent. Reaction of fused oxazinones with amines has been broadly described in the literature.

### Reaction Scheme 3: Preparation of fused oxazinones of Formula IV:

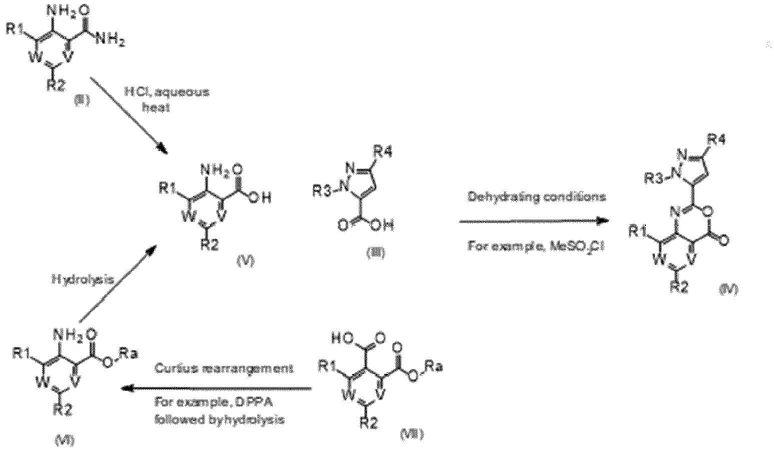

Fused oxazinones of formula IV, wherein R₁, R₂, R₃, R₄, V and Ware as defined in formula I, can be obtained by condensing a pyrazole carboxylic acid of formula III, wherein R₃ and R₄ are as defined in formula I, with ortho-amino heteroarylcarboxylic acid of formula V, wherein R₁, R₂, V and W are as defined in formula I. Frequently used intermolecular dehydration conditions consist of treatment of the mixture of the components with, for example, methanesulfonyl chloride, usually in presence of a base, like pyridine and in a solvent like, for example, acetonitrile. The amino acid of formula V can be obtained by hydrolysis (usually HCl, aqueous) of the amino-amide of formula II or through alkaline hydrolysis of the ortho-amino ester of formula VI, wherein R₁, R₂, V and Ware as defined in formula I, and Ra is a C1-C3-alkyl group. The amino ester can be obtained from dicarboxylic acid monoalkyl ester via Curtius rearrangement. A very popular reaction sequence to perform this transformation is the treatment of the carboxylic acid with diphenoxy phosphoryl azide followed by thermal rearrangement and hydrolysis of the intermediate isocyanate. If the reaction is performed in an alcohol (for example t-butanol), the corresponding carbamate must be hydrolyzed.

Many pyrazole carboxylic acids of formula III are described in the literature and compounds of formula III can be prepared as already described or in a similar way by a person skilled in the art (for example in WO04/067528, WO10069502, WO 2011/157664, WO04046129). Some of them are also commercially available.

Compounds of formula IIIb, in which R₃ has the meaning given for formula I and Y0 is Y as defined for formula I are generally known or can be prepared by those skilled in the art. A typical example of such a synthesis is shown in Scheme 4.

### Reaction Scheme 4: Preparation of selected compounds of Formula III

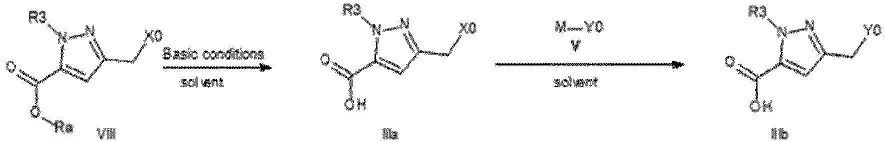

For example, compounds of formula IIIb may be prepared by reaction between compounds of formula V, wherein Y0 is defined as above and M is an alkali metal such as lithium, sodium, potassium, and compounds of formula IIIa, wherein R₃ has the meaning given for formula I and X0 is chlorine, bromine or iodine, in suitable solvents that may include, for example, acetone or tetrahydrofuran, optionnaly in the presence of potassium iodide as catalyst.

Compounds of formula IIIa, wherein R₃ has the meaning given for formula I and X0 is chlorine, bromine or iodine, can be prepared from compounds of formula VIII, wherein R₃ has the meaning given for formula I, Ra is a C₁-C₃alkyl group and X0 is chlorine, bromine or iodine, under basic conditions that may include, for example lithium hydroxide or sodium hydroxide, in suitable solvents such as, tetrahydrofuran, methanol or dioxane. Such processes have been described previously, for example, in WO2011073101. Compounds of formula VIII are generally known or can be easily prepared by those skilled in the art. A typical example of such a synthesis is described in WO2011073101.

Depending on the procedure or the reaction conditions, the reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reactions are advantageously carried out in a temperature range from approximately -80°C to approximately +140°C, preferably from approximately -30°C to approximately +100°C, in many cases in the range between ambient temperature and approximately +80°C.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula I can be prepared in a manner known *per se.* Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known per se into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula I represented in Tables A-1 to A-7, B-1 to B-7 and C-1 to C-7 can be prepared according to the methods described above. The examples which follow are intended to illustrate the invention and show preferred compounds of formula I.

**Table X**

| **Compound number** | **R1** | **R2** |
|---|---|---|
| 1 | Cl | Cl |
| 2 | Cl | Br |
| 3 | Br | Cl |
| 4 | Br | Br |
| 5 | CH₃ | Cl |
| 6 | CH₃ | Br |

Table A-1: provides 6 compounds A-1.001 to A-1.006 of formula Xa1 where R1 and R2 are as defined in Table X.

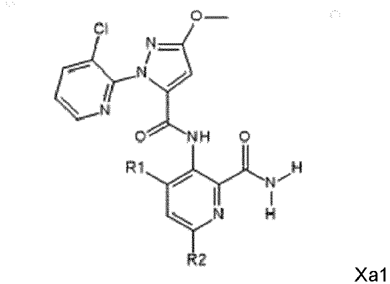

Table A-2: provides 6 compounds A-2.001 to A-2.006 of formula Xa2 where R1 and R2 are as defined in Table X.

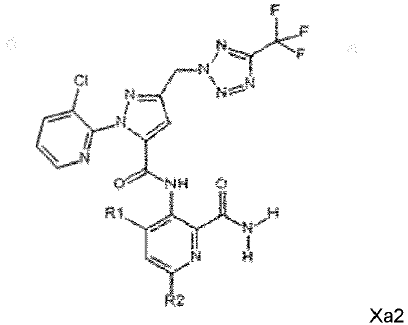

Table A-3: provides 6 compounds A-3.001 to A-3.006 of formula Xa3 where R1 and R2 are as defined in Table X.

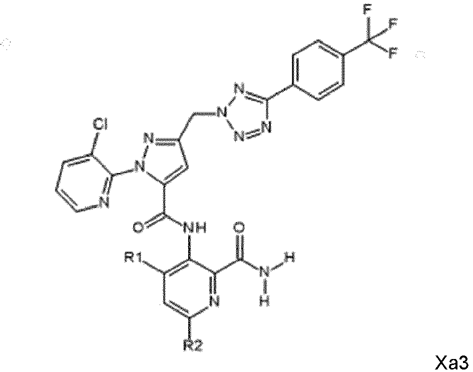

Table A-4: provides 6 compounds A-4.001 to A-4.006 of formula Xa4 where R1 and R2 are as defined in Table X.

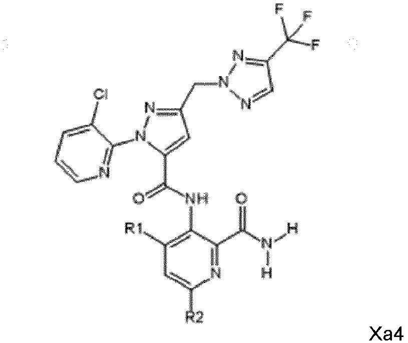

Table A-5: provides 6 compounds A-5.001 to A-5.006 of formula Xa5 where R1 and R2 are as defined in Table X.

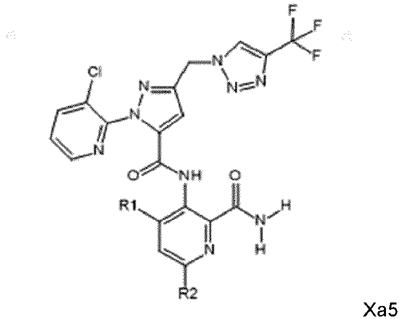

Table A-6: provides 6 compounds A-6.001 to A-6.006 of formula Xa6 where R1 and R2 are as defined in Table X.

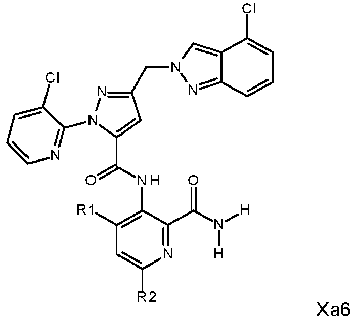

Table A-7: provides 6 compounds A-7.001 to A-7.006 of formula Xa7 where R1 and R2 are as defined in Table X.

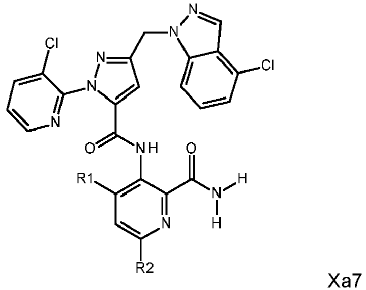

Table B-1: provides 6 compounds B-1.001 to B-1.006 of formula Xb1 where R1 and R2 are as defined in Table X.

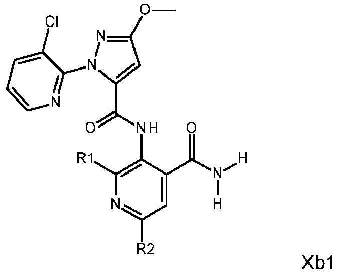

Table B-2: provides 6 compounds B-2.001 to B-2.006 of formula Xb2 where R1 and R2 are as defined in Table X.

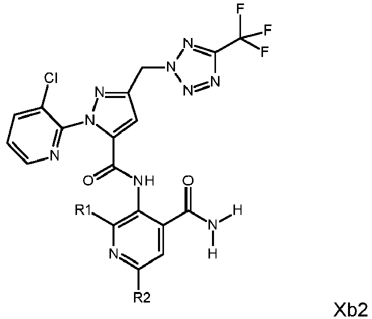

Table B-3: provides 6 compounds B-3.001 to B-3.006 of formula Xb3 where R1 and R2 are as defined in Table X.

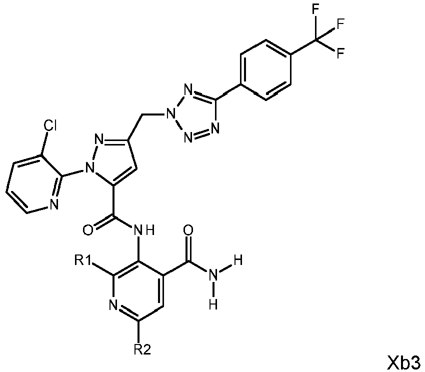

Table B-4: provides 6 compounds B-4.001 to B-4.006 of formula Xb4 where R1 and R2 are as defined in Table X.

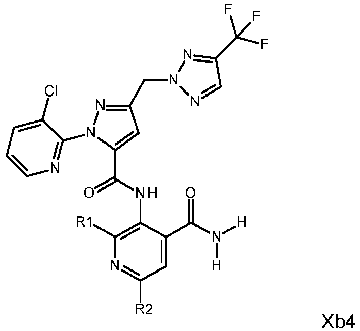

Table B-5: provides 6 compounds B-5.001 to B-5.006 of formula Xb5 where R1 and R2 are as defined in Table X.

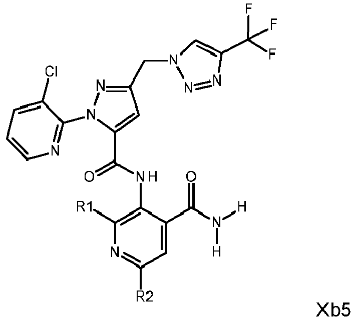

Table B-6: provides 6 compounds B-6.001 to B-6.006 of formula Xb6 where R1 and R2 are as defined in Table X.

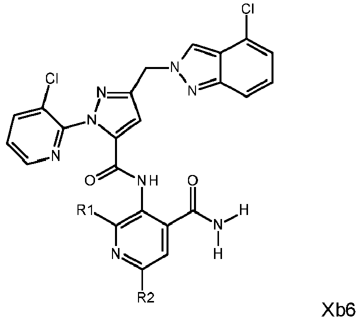

Table B-7: provides 6 compounds B-7.001 to B-7.006 of formula Xb7 where R1 and R2 are as defined in Table X.

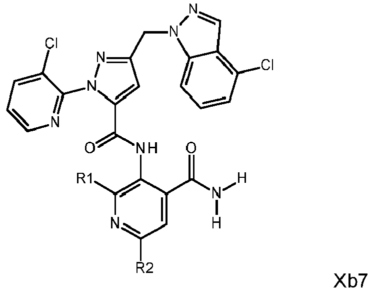

Table C-1: provides 6 compounds C-1.001 to C-1.006 of formula Xc1 where R1 and R2 are as defined in Table X.

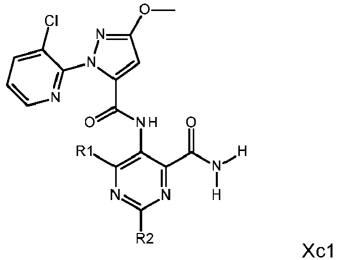

Table C-2: provides 6 compounds C-2.001 to C-2.006 of formula Xc2 where R1 and R2 are as defined in Table X.

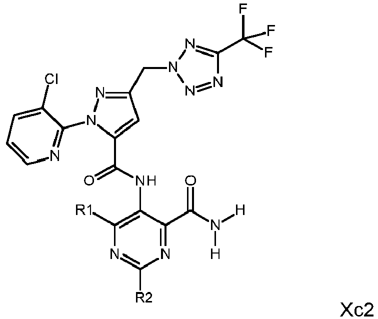

Table C-3: provides 6 compounds C-3.001 to C-3.006 of formula Xc3 where R1 and R2 are as defined in Table X.

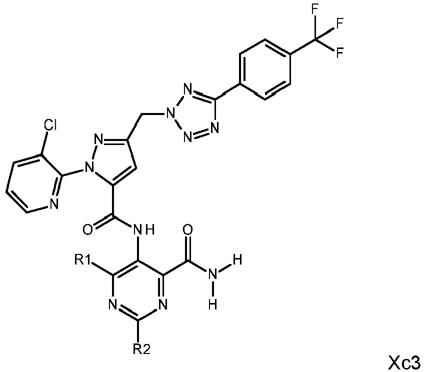

Table C-4: provides 6 compounds C-4.001 to C-4.006 of formula Xc4 where R1 and R2 are as defined in Table X.

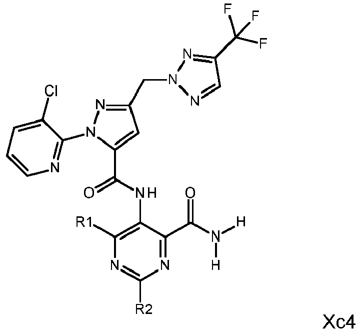

Table C-5: provides 6 compounds C-5.001 to C-5.006 of formula Xc5 where R1 and R2 are as defined in Table X.

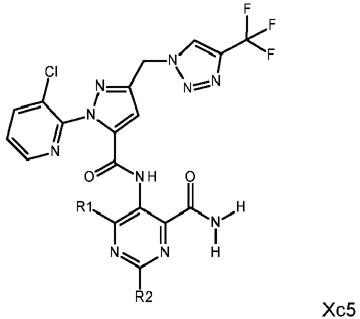

Table C-6: provides 6 compounds C-6.001 to C-6.006 of formula Xc6 where R1 and R2 are as defined in Table X.

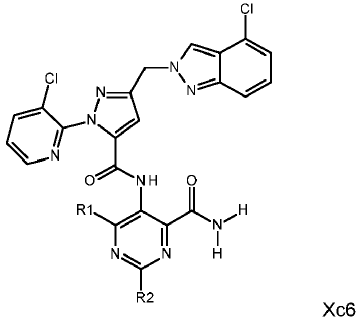

Table C-7: provides 6 compounds C-7.001 to C-7.006 of formula Xc7 where R1 and R2 are as defined in Table X.

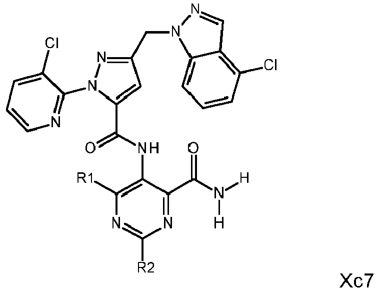

Also made available are certain intermediate compounds of formula IV, some of which are novel. For example,
- a compound of formula IV, where W, V, R₁, R₂, and R₃ are as defined in the first aspect, and R₄ is C₁-C₃haloalkyl, C₁-C₃alkoxy, C₁-C₃haloalkoxy, or CH₂-Y (where Y is Ya1 to Ya6); or
- a compound of formula IV, where W and V are as defined in the first aspect, R₁ and R₂ are as defined in Table X, and R₃ are as defined in the first aspect, and R₄ is C₁-C₃haloalkyl, C₁-C₃alkoxy, C₁-C₃haloalkoxy, or CH₂-Y (where Y is Ya1 to Ya6); or
- a compound of formula IV, where W and V are as defined in the first aspect, R₁ and R₂ are as defined in Table X, and R₃ are as defined in the first aspect, and R₄ is C₁-C₃alkoxy, C₁-C₃haloalkoxy, or CH₂-Y (where Y is Ya1 to Ya6); or
- a compound of formula IV, where W, V, and R₃ are as defined in the first aspect, R₁ and R₂ are each chlorine, and R₄ is C₁-C₃alkoxy, C₁-C₃haloalkoxy, or CH₂-Y (where Y is Ya1 to Ya6); or
- a compound of formula IV, where W, V, are as defined in the first aspect, R₁ and R₂ are each chlorine, R₃ is 3-chloro-pyrid-2-yl ring or 3,5-dichloro-pyrid-2-yl ring, and R₄ is C₁-C₃alkoxy, C₁-C₃haloalkoxy, or CH₂-Y (where Y is Ya1 to Ya6); or
- a compound of formula IV, where W is CH, V is N, R₁ and R₂ are each chlorine, R₃ is 3-chloro-pyrid-2-yl ring or 3,5-dichloro-pyrid-2-yl ring, and R₄ is C₁-C₃alkoxy, C₁-C₃haloalkoxy, or CH₂-Y (where Y is Ya1 to Ya6); or
- a compound of formula IV, where W is CH, V is N, R₁ and R₂ are each chlorine, R₃ is 3-chloro-pyrid-2-yl ring, and R₄ is trifluoromethyl, 1,1,1-trifluoroethoxy, difluoromethoxy, methoxy, or CH₂-Y (where Y is Ya1 to Ya6); or
- a compound of formula IV, where W is N, V is N, R₁ and R₂ are each chlorine, R₃ is 3-chloro-pyrid-2-yl ring, and R₄ is trifluoromethyl, 1,1,1-trifluoroethoxy, difluoromethoxy, methoxy, or CH₂-Y (where Y is Ya1 to Ya6).

The compounds of formula I according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate.

Examples of the above mentioned animal pests are:
from the order *Acarina,* for example,
   Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura*, for example,
   Haematopinus spp., Linognathus spp., Pediculus spp., Pemphigus spp. and Phylloxera spp.;
from the order *Coleoptera*, for example,
   Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemlineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.; from the order *Diptera,* for example,
   Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
   Acanthocoris scabrator, Acrosternum spp, Adelphocoris lineolatus, Aleurodes spp., Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euchistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
   Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
   Acromyrmex, Arge spp, Atta spp., Cephus spp., Diprion spp., Diprionidae, Gilpinia polytoma, Hoplocampa spp., Lasius spp., Monomorium pharaonis, Neodiprion spp., Pogonomyrmex spp, Slenopsis invicta, Solenopsis spp. and Vespa spp.;
from the order *Isoptera,* for example,
   Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example,
   Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Elasmopalpus lignosellus, Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
   Damalinea spp. and Trichodectes spp.;
from the order *Orthoptera,* for example,
   Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera*, for example,
   Liposcelis spp.;
from the order *Siphonaptera*, for example,
   Ceratophyllus spp., Ctenocephalides spp. and Xenopsylla cheopis;
from the order *Thysanoptera*, for example,
   Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target plant or crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. B. *elatior*, *B. semperflorens*, *B. tubéreux*), *Bougainvillea* spp., *Brachycome* spp., *Brassica* spp. (ornamental), *Calceolaria* spp., *Capsicum annuum*, *Catharanthus roseus*, *Canna* spp., *Centaurea* spp., *Chrysanthemum* spp., *Cineraria* spp. (C. *maritime*), *Coreopsis* spp., *Crassula coccinea*, *Cuphea ignea*, *Dahlia* spp., *Delphinium* spp., *Dicentra spectabilis*, *Dorotheantus* spp., *Eustoma grandiflorum, Forsythia* spp., *Fuchsia* spp., *Geranium gnaphalium*, *Gerbera* spp., *Gomphrena globosa*, *Heliotropium* spp., *Helianthus* spp., *Hibiscus* spp., *Hortensia* spp., *Hydrangea* spp., *Hypoestes phyllostachya*, *Impatiens* spp. (*I*. *Walleriana*), *Iresines* spp., *Kalanchoe* spp., *Lantana camara*, *Lavatera trimestris*, *Leonotis leonurus*, *Lilium* spp., *Mesembryanthemum* spp., *Mimulus* spp., *Monarda* spp., *Nemesia* spp., *Tagetes* spp., *Dianthus* spp. (carnation), Canna spp., *Oxalis* spp., *Bellis* spp., *Pelargonium* spp. (*P. peltatum, P. Zonale*), *Viola* spp. (pansy), *Petunia* spp., *Phlox* spp., *Plecthranthus* spp., *Poinsettia* spp., *Parthenocissus* spp. (*P. quinquefolia, P. tricuspidata*), *Primula* spp., *Ranunculus* spp., *Rhododendron* spp., *Rosa* spp. (rose), *Rudbeckia* spp., *Saintpaulia* spp., *Salvia* spp., *Scaevola aemola, Schizanthus wisetonensis*, *Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. (*A*. *sativum, A*. *cepa, A. oschaninii, A. Porrum, A. ascalonicum, A. fistulosum*)*, Anthriscus cerefolium, Apium graveolus, Asparagus officinalis, Beta vulgarus, Brassica* spp. (*B*. *Oleracea, B. Pekinensis, B. rapa), Capsicum annuum, Cicer arietinum, Cichorium endivia, Cichorum* spp. (C. *intybus, C. endivia), Citrillus lanatus, Cucumis* spp. (C. *sativus, C. melo*)*, Cucurbita* spp. (C. *pepo, C. maxima), Cyanara* spp. (C. *scolymus, C. cardunculus*)*, Daucus carota, Foeniculum vulgare, Hypericum* spp., *Lactuca sativa, Lycopersicon* spp. *(L. esculentum, L. lycopersicum), Mentha* spp., *Ocimum basilicum, Petroselinum crispum, Phaseolus* spp. (P. *vulgaris, P. coccineus), Pisum sativum, Raphanus sativus, Rheum rhaponticum, Rosemarinus* spp., *Salvia* spp., *Scorzonera hispanica, Solanum melongena, Spinacea oleracea, Valerianella* spp. (*V*. *locusta, V. eriocarpa)* and *Vicia faba.*

Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

The compounds of formula I are particularly suitable for control of
- a pest of the order Lepidoptera, for example, one or more of the species Spodoptera littoralis, Spodoptera frugiperda, Plutella xylostella, Cnaphalocrocis medinalis, Cydia pomonella, Chrysodeixis includens, Chilo suppressalis, Elasmopalpus lignosellus, Pseudoplusia includens, and Tuta absoluta (preferably in vegetables and corn).

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (O*strinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de I'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention provides a compound of the second aspect for use in therapy. The present invention provides a compound of the first aspect, for use in controlling parasites in or on an animal. The present invention further provides a compound of the first aspect, for use in controlling ectoparasites on an animal. The present invention further provides a compound of the first aspect, for use in preventing and/or treating diseases transmitted by ectoparasites.

The present invention provides the use of a compound of the second aspect, for the manufacture of a medicament for controlling parasites in or on an animal. The present invention further provides the use of a compound of the first aspect, for the manufacture of a medicament for controlling ectoparasites on an animal. The present invention further provides the use of a compound of the first aspect, for the manufacture of a medicament for preventing and/or treating diseases transmitted by ectoparasites.

The present invention provides the use of a compound of the first aspect, in controlling parasites in or on an animal. The present invention further provides the use of a compound of the second aspect, in controlling ectoparasites on an animal.

The term "controlling" when used in context of parasites in or on an animal refers to reducing the number of pests or parasites, eliminating pests or parasites and/or preventing further pest or parasite infestation.

The term "treating" when used used in context of parasites in or on an animal refers to restraining, slowing, stopping or reversing the progression or severity of an existing symptom or disease.
The term "preventing" when used used in context of parasites in or on an animal refers to the avoidance of a symptom or disease developing in the animal.

The term "animal" when used used in context of parasites in or on an animal may refer to a mammal and a non-mammal, such as a bird or fish. In the case of a mammal, it may be a human or non-human mammal. Non-human mammals include, but are not limited to, livestock animals and companion animals. Livestock animals include, but are not limited to, cattle, camellids, pigs, sheep, goats and horses. Companion animals include, but are not limited to, dogs, cats and rabbits.

A "parasite" is a pest which lives in or on the host animal and benefits by deriving nutrients at the host animal's expense. An "endoparasite" is a parasite which lives in the host animal. An "ectoparasite" is a parasite which lives on the host animal. Ectoparasites include, but are not limited to, acari, insects and crustaceans (e.g. sea lice). The Acari (or Acarina) sub-class comprises ticks and mites. Ticks include, but are not limited to, members of the following genera: *Rhipicaphalus,* for example, *Rhipicaphalus* (*Boophilus*) *microplus* and *Rhipicephalus sanguineus*; *Amblyomrna*; *Dermacentor*; *Haemaphysalis*; *Hyalomma*; *Ixodes*; *Rhipicentor*; *Margaropus*; *Argas*; *Otobius*; and *Ornithodoros.* Mites include, but are not limited to, members of the following genera: *Chorioptes,* for example *Chorioptes bovis*; *Psoroptes,* for example *Psoroptes ovis*; *Cheyletiella*; *Dermanyssus*; for example *Dermanyssus gallinae*; *Ortnithonyssus*; *Demodex*, for example *Demodex canis*; *Sarcoptes*, for example *Sarcoptes scabiei*; and *Psorergates.* Insects include, but are not limited to, members of the orders: Siphonaptera, Diptera, Phthiraptera, Lepidoptera, Coleoptera and Homoptera. Members of the Siphonaptera order include, but are not limited to, *Ctenocephalides felis* and *Ctenocephatides canis.* Members of the Diptera order include, but are not limited to, *Musca spp.*; bot fly, for example *Gasterophilus intestinalis* and *Oestrus ovis*; biting flies; horse flies, for example *Haematopota spp.* and *Tabunus spp.*; *haematobia*, for example *haematobia irritans*; *Stomoxys*; *Lucilia*; midges; and mosquitoes. Members of the Phthiraptera class include, but are not limited to, blood sucking lice and chewing lice, for example *Bovicola Ovis* and *Bovicola Bovis.*

The term "effective amount" when used used in context of parasites in or on an animal refers to the amount or dose of the compound of the invention, or a salt thereof, which, upon single or multiple dose administration to the animal, provides the desired effect in or on the animal. The effective amount can be readily determined by the attending diagnostician, as one skilled in the art, by the use of known techniques and by observing results obtained under analogous circumstances. In determining the effective amount a number of factors are considered by the attending diagnostician, including, but not limited to: the species of mammal; its size, age, and general health; the parasite to be controlled and the degree of infestation; the specific disease or disorder involved; the degree of involvement or the severity of the disease or disorder; the response of the individual; the particular compound administered; the mode of administration; the bioavailability characteristics of the preparation administered; the dose regimen selected; the use of concomitant medication; and other relevant circumstances.

The compounds of the invention may be administered to the animal by any route which has the desired effect including, but not limited to topically, orally, parenterally' and subcutaneously. Topical administration is preferred. Formulations suitable for topical administration include, for example, solutions, emulsions and suspensions and may take the form of a pour-on, spot-on, spray-on, spray race or dip. In the alternative, the compounds of the invention may be administered by means of an ear tag or collar.

Salt forms of the compounds of the invention include both pharmaceutically acceptable salts and veterinary acceptable salts, which can be different to agrochemically acceptable salts. Pharmaceutically and veterinary acceptable salts and common methodology for preparing them are well known in the art. See, for example, Gould, P.L., "Salt selection for basic drugs", International Journal of Pharmaceutics, 33: 201 -217 (1986); Bastin, R.J., et al. "Salt Selection and Optimization Procedures for Pharmaceutical New Chemical Entities", Organic Process Research and Development, 4: 427-435 (2000); and Berge, S.M., et al., "Pharmaceutical Salts", Journal of Pharmaceutical Sciences, 66: 1-19, (1977). One skilled in the art of synthesis will appreciate that the compounds of the invention are readily converted to and may be isolated as a salt, such as a hydrochloride salt, using techniques and conditions well known to one of ordinary skill in the art. In addition, one skilled in the art of synthesis will appreciate that the compounds of the invention are readily converted to and may be isolated as the corresponding free base from the corresponding salt.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO 2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera*, especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs, ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, C. *lurida*), *Rhizotrogus spp.* (e.g. European chafer, *R. majalis*), *Cotinus spp.* (e.g. Green June beetle, C. *nitida*), *Popillia spp.* (e.g. Japanese beetle, *P. japonica*), *Phyllophaga spp.* (e.g. May/June beetle), *Ataenius spp.* (e.g. Black turfgrass ataenius, *A. spretulus*), *Maladera spp.* (e.g. Asiatic garden beetle, M. *castanea*) and *Tomarus spp*.), ground pearls (*Margarodes* spp.), mole crickets (tawny, southern, and short-winged; *Scapteriscus* spp., *Gryllotalpa africana*) and leatherjackets (European crane fly, *Tipula spp.*)*.*

The present invention may also be used to control insect pests of turfgrass that are thatch dwelling, including armyworms (such as fall armyworm *Spodoptera frugiperda*, and common armyworm *Pseudaletia unipuncta*), cutworms, billbugs (*Sphenophorus spp.,* such as S. *venatus verstitus* and S. *parvulus),* and sod webworms (such as *Crambus spp.* and the tropical sod webworm, *Herpetogramma phaeopteralis).*

The present invention may also be used to control insect pests of turfgrass that live above the ground and feed on the turfgrass leaves, including chinch bugs (such as southern chinch bugs, *Blissus insularis),* Bermudagrass mite (*Eriophyes cynodoniensis),* rhodesgrass mealybug (*Antonina graminis),* two-lined spittlebug (*Propsapia bicincta)*, leafhoppers, cutworms (*Noctuidae* family), and greenbugs.

The present invention may also be used to control other pests of turfgrass such as red imported fire ants (*Solenopsis invicta)* that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention may be also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds of formula I, or salts thereof, are especially suitable for controlling one or more pests selected from order Lepidoptera, especially one or more of the species Spodoptera littoralis, Spodoptera frugiperda, Plutella xylostella, Cnaphalocrocis medinalis, Cydia pomonella, Chrysodeixis includens, Chilo suppressalis, Elasmopalpus lignosellus, Pseudoplusia includens, and Tuta absoluta (preferably in vegetables and corn).. In a preferred embodiment of each aspect, a compound TQ (where the abbreviation "TQ" means "one compound selected from the compounds defined in List Q") controls one or more of pests selected from the species Spodoptera littoralis, Spodoptera frugiperda, Plutella xylostella, Cnaphalocrocis medinalis, Cydia pomonella, Chrysodeixis includens, Chilo suppressalis, Elasmopalpus lignosellus, Pseudoplusia includens, and Tuta absoluta (preferably in vegetables and corn).

The compounds of formula I, or salts thereof, are especially suitable for controlling one or more of the insects having diamide resistance selected from: Spodoptera littoralis, Spodoptera frugiperda, Plutella xylostella, Cnaphalocrocis medinalis, Cydia pomonella, Chrysodeixis includens, Chilo suppressalis, Elasmopalpus lignosellus, Pseudoplusia includens, and Tuta absoluta. In a preferred embodiment of each aspect, a compound TQ (where the abbreviation "TQ" means "one compound selected from the compounds defined in List Q") controls one or more of the insects having diamide resistance selected from: Spodoptera littoralis, Spodoptera frugiperda, Plutella xylostella, Cnaphalocrocis medinalis, Cydia pomonella, Chrysodeixis includens, Chilo suppressalis, Elasmopalpus lignosellus, Pseudoplusia includens, and Tuta absoluta.

The compounds of formula I, or salts thereof, are especially suitable for controlling one or more of insects having diamide resistance selected from: Plutella xylostella, Chilo suppressalis, and Tuta absoluta.

In a preferred embodiment of each aspect, a compound TQ (where the abbreviation "TQ" means "one compound selected from the compounds defined in List Q") controls one or more insect selected from Plutella xylostella, Chilo suppressalis, and Tuta absoluta, which insect demonstrates resistance against IRAC MoA Group 28 insecticides, such as Plutella xylostella + TQ, Chilo suppressalis + TQ, and Tuta absoluta + TQ.

Compounds according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile (against non-target organisms above and below ground (such as fish, birds and bees), improved physico-chemical properties, or increased biodegradability). In particular, it has been surprisingly found that certain compounds of formula I may show an advantageous safety profile with respect to non-target arthropods, in particular pollinators such as honey bees, solitary bees, and bumble bees. Most particularly, Apis mellifera.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 I/ha, especially from 10 to 1000 I/ha.

Preferred formulations can have the following compositions (weight %):

| Emulsifiable concentrates: | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

| Dusts: | |
|---|---|
| active ingredient: | 0.1 to 10 %, preferably 0.1 to 5 % |
| solid carrier: | 99.9 to 90 %, preferably 99.9 to 99 % |

| Suspension concentrates: | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

| Wettable powders: | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

| Granules: | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25% | 50% | 75 % |
| sodium lignosulfonate | 5% | 5 % | - |
| sodium lauryl sulfate | 3% | - | 5% |
| sodium diisobutylnaphthalenesulfonate | - | 6% | 10% |
| phenol polyethylene glycol ether (7-8 mol of ethylene oxide) | - | 2 % | - |
| highly dispersed silicic acid | 5% | 10% | 10% |
| Kaolin | 62% | 27% | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25% | 50% | 75 % |
| light mineral oil | 5% | 5% | 5% |
| highly dispersed silicic acid | 5% | 5% | - |
| Kaolin | 65% | 40% | - |
| Talcum | - | | 20% |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3% |
| calcium dodecylbenzenesulfonate | 3% |
| castor oil polyglycol ether (35 mol of ethylene oxide) | 4% |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5% | 6% | 4% |
| Talcum | 95 % | - | - |
| Kaolin | - | 94% | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15% |
| sodium lignosulfonate | 2% |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8% |
| polyethylene glycol (mol. wt. 200) | 3% |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6% |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5% |
| copolymer butanol PO/EO | 2% |
| Tristyrenephenole with 10-20 moles EO | 2% |
| 1,2-benzisothiazolin-3-one (in the form of a 20% solution in water) | 0.5 % |
| monoazo-pigment calcium salt | 5% |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹ H NMR measurements were recorded on a Brucker 400MHz spectrometer, chemical shifts are given in ppm relevant to a TMS standard. Spectra measured in deuterated solvents as indicated. Either one of the LC-MS methods below was used to characterize the compounds. The characteristic LC-MS values obtained for each compound were the retention time ("Rt", recorded in minutes) and the measured molecular ion (M+H)⁺.

### LC-MS and GC-MS Methods:

LC-MS standard:
Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 l/h, Desolvation Gas Flow: 650-1000 l/h, Mass range: 100 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 1.5 min; Solvents: A = water + 5% MeOH + 0.05 % HCOOH, B= Acetonitrile + 0.05 % HCOOH; Flow (ml/min) 0.85, Gradient: 10% B isocratic for 0.2 min, then 10-100% B in 1.0 min, 100% B isocratic for 0.2min, 100-10% B in 0.05min, 10% B isocratic for 0.05 min.

LC-MS standard long:
Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 l/h, Desolvation Gas Flow: 650-1000 l/h, Mass range: 100 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 3.0 min; Solvents: A = water + 5% MeOH + 0.05 % HCOOH, B= Acetonitrile + 0.05 % HCOOH; Flow (ml/min) 0.85, Gradient: 10% B isocratic for 0.2 min, then 10-100% B in 2.5 min, 100% B isocratic for 0.3min.

### GC-MS Method 3:

GC-MS was conducted on a Thermo, MS: ISQ and GC: Trace GC 1310 with a column from Zebron phenomenex: Phase ZB-5ms 15 m, diam: 0.25 mm, 0.25 µm, He flow 1.5 ml/min, temp injector: 250°C, temp detector: 220°C, method: hold 0.7min at 60 °C, 80°C/min until 320°C, hold 2 min at 320°C, total time 6min. Cl reagent gas: Methane, flow 1ml/min, ionization mode Cl, polarity positive, scan time 0.2 sec, Scan mass range 50-650amu

### LC-MS Method 4:

Spectra were recorded on a Mass Spectrometer from Agilent (Single quad mass spectrometer) equipped with an Multimode- Electron Spray and APCI (Polarity: positive and negative ions), Capillary: 4.00KV, Corona Current 4.0µA, Charging Voltage, 2.00kV, Nitrogen Gas Flow:9.0L/min, Nebulizer Pressure: 40psig, Mass range: 100 to 1000 m/z), dry gas temperature 250°C,Vaporizer temperature 200°C and Spectra were recorded on LCMS from Agilent: quaternary pump, heated column compartment, Variable wave length detector. Column: Eclipse XDB C18, 5.0 µm, 150x4.6 mm, column Temp: Ambient, Wavelength (nm): 220nm, Solvents: A =0.05% TFA in water, B = 0.05% TFA in Acetonitrile. Gradient: time/%B: 0/5, 0.5/5, 3.5/90, 5/90, 5.1/5, 7/5; Flow rate: 1.0ml/min.

### Example 1: Preparation of 4,6-dichloro-3-[[2-(3-chloro-2-pyridyl)-5-(2,2,2-trifluoroethoxy) pyrazole-3-carbonyl]amino]pyridine-2-carboxamide (Compound P.2)

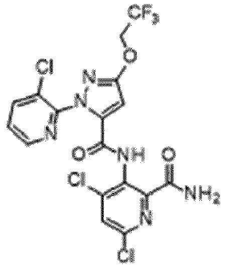

Under argon, to a solution of methane sulfonyl chloride (0.0773 mL, 0.976 mmol, 2.00 equiv.) in acetonitrile (1.0 mL) were added dropwise at 0 °C a solution of 2-(3-chloro-2-pyridyl)-5-(2,2,2-trifluoroethoxy)pyrazole-3-carboxylic acid (0.165 g, 0.488 mmol, 1.00 equiv.) in acetonitrile (2.0 mL) and pyridine (0.040 mL). The reaction mixture was stirred at 0 °C for 30 minutes. Then, a suspension of 3-amino-4,6-dichloro-pyridine-2-carboxylic acid (0.101 g, 0.488 mmol, 1.00 equiv.) in acetonitrile (2.0 mL) was added to the previous solution at 0 °C, followed by pyridine (0.080 mL). The reaction mixture was allowed to reach room temperature and it was stirred at room temperature overnight. Ammonia (0.4 M in dioxane, 6.00 mL, 2.93 mmol, 6.00 equiv.) was added at room temperature and it was stirred for 1 hour. The reaction mixture was extracted with ethyl acetate, washed with water, then brine, dried over magnesium sulfate, filtered and concentrated under reduced pressure. Dichloromethane was added to the residue and the insoluble precipitate was filtered off. Purification of the filtrate by flash chromatography (ethyl acetate in cyclohexane) afforded the desired product 4,6-dichloro-3-[[2-(3-chloro-2-pyridyl)-5-(2,2,2-trifluoroethoxy)pyrazole-3-carbonyl]amino]pyridine-2-carboxamide.

LC-MS (method standard): retention time 0.99 min, m/z 511 [M+H⁺],

¹H NMR (400 MHz, chloroform-d) δ ppm 11.12 (s, 1 H) 8.48 (dd, J=4.72, 1.45 Hz, 1 H) 7.85 (dd, J=7.99, 1.45 Hz, 1 H) 7.73 - 7.81 (m, 1 H) 7.55 (s, 1 H) 7.37 (dd, J=7.99, 4.72 Hz, 1 H) 6.60 (s, 1 H) 5.69 (br s, 1 H) 4.70 (q, J=8.23 Hz, 2 H)

¹⁹F NMR (377 MHz, chloroform-d) δ ppm -74.26 (s, CF₃).

**Table P: Physical data of compounds of formula I**

| Entry | IUPAC name | STRUCTURE | RT (min) | [M+H] (measured) | Method | ¹H-NMR | ¹⁹F-NMR |
|---|---|---|---|---|---|---|---|
| P.1 | 4,6-dichloro-3-[[2-(3-chloro-2-pyridyl)-5-(trifluoromethyl)pyrazole-3-carbonyl]amino]pyridine-2-carboxamide | 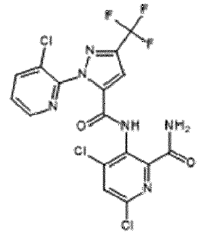 | 0.98 | 481/483/485 | Standard | 1H NMR (400 MHz, CHLOROFORM-d) δ ppm 11.26 (s, 1 H) 8.51 (dd, J=4.72, 1.45 Hz, 1 H) 7.90 (dd, J=8.36, 1.45 Hz, 1 H) 7.75 - 7.85 (m, 1 H) 7.57 (s, 1 H) 7.44 (dd, J=7.99, 4.72 Hz, 1 H) 7.32 (s, 1 H) 5.72 (br s, 1 H) | 19F NMR (377 MHz, CHLOROFORM-d) δ ppm -62.35 (s, CF3) |
| P.2 | 4,6-dichloro-3-[[2-(3-chloro-2-pyridyl)-5-(2,2,2-trifluoroethoxy)pyrazole-3-carbonyl]amino]pyridine-2-carboxamide | 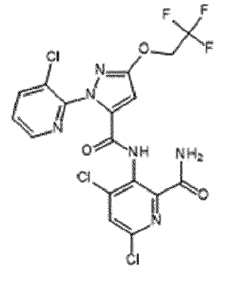 | 0.99 | 511/513/515 | Standard | 1H NMR (400 MHz, CHLOROFORM-d) δ ppm 11.12 (s, 1 H) 8.48 (dd, J=4.72, 1.45 Hz, 1 H) 7.85 (dd, J=7.99, 1.45 Hz, 1 H) 7.73 - 7.81 (m, 1 H) 7.55 (s, 1 H) 7.37 (dd, J=7.99, 4.72 Hz, 1 H) 6.60 (s, 1 H) 5.69 (br s, 1 H) 4.70 (q, J=8.23 Hz, 2 H) | 19F NMR (377 MHz, CHLOROFORM-d) δ ppm -74.26 (s, CF3) |
| P.3 | 4,6-dichloro-3-[[2-(3-chloro-2-pyridyl)-5-(difluoromethoxy)pyrazole-3-carbonyl]amino]pyridine-2-carboxamide | 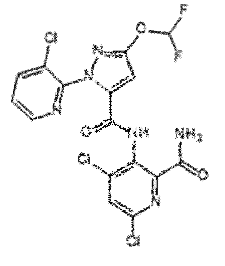 | 0.93 | 479/481/483 | Standard | 1H NMR (400 MHz, CHLOROFORM-d) δ ppm 11.16 (s, 1 H) 8.48 (dd, J=4.72, 1.45 Hz, 1 H) 7.87 (dd, J=7.99, 1.45 Hz, 1 H) 7.74 - 7.82 (m, 1 H) 7.56 (s, 1 H) 7.39 (dd, J=7.99, 4.72 Hz, 1 H) 7.01 (t, 1 H) 6.72 (s, 1 H) 5.72 (br s, 1 H) | 19F NMR (377 MHz, CHLOROFORM-d) δ ppm -84.78 (s, CHF2) |
| P.4 | 4,6-dichloro-3-[[2-(3-chloro-2-pyridyl)-5-methoxy-pyrazole-3-carbonyl]amino]pyridin e-2-carboxamide | 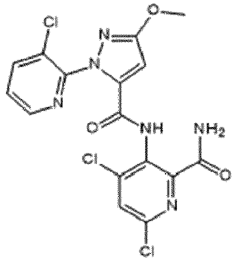 | 0.85 | 443/445/447 | Standard | 1H NMR (400 MHz, CHLOROFORM-d) δ ppm 11.05 (s, 1 H) 8.48 (dd, J=4.72, 1.45 Hz, 1 H) 7.83 (dd, J=7.99, 1.45 Hz, 1 H) 7.73 - 7.79 (m, 1 H) 7.54 (s, 1 H) 7.34 (dd, J=7.99, 4.72 Hz, 1 H) 6.51 (s, 1 H) 5.71 (br s, 1 H) 4.02 (s, 3 H) | |

**Table I: Intermediates**

| Entry | IUPAC name | STRUCTURE | RT (min) | [M+H] measured | Method | | |
|---|---|---|---|---|---|---|---|
| I 1 | 6,8-dichloro-2-[2-(3-chloro-2-pyridyl)-5-(trifluoromethyl) pyrazol-3-yl]pyrido[3,2-d][1,3]oxazin-4-one | 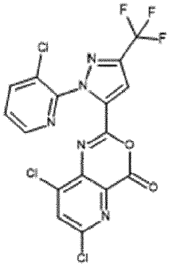 | 1.09 | 462/464/466 | Standard | | |
| I 2 | 6,8-dichloro-2-[2-(3-chloro-2-pyridyl)-5-(2,2,2-trifluoroethoxy)pyrazol-3-yl]pyrido[3,2-d][1,3]oxazin-4-one | 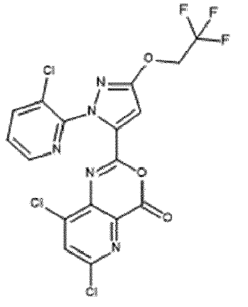 | 1.10 | 492/494/496 | Standard | | |
| I.3 | 6,8-dichloro-2-[2-(3-chloro-2-pyridyl)-5-(difluoromethoxy) pyrazol-3-yl]pyrido[3,2-d][1,3]oxazin-4-one | 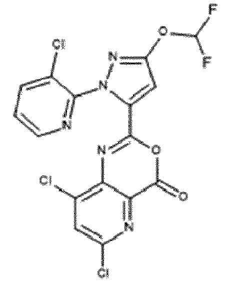 | 1.05 | 460/462/464 | Standard | | |
| I.4 | 6,8-dichloro-2-[2-(3-chloro-2-pyridyl)-5-methoxy-pyrazol-3-yl]pyrido[3,2-d][1,3]oxazin-4-one | 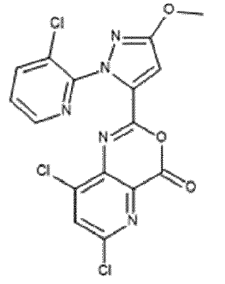 | 0.99 | 424/426/428 | Standard | | |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of a compound of formula I with an active substance are preferred (the abbreviation "TX" means "one compound selected from the compounds defined in Tables A-1 to A-7, B-1- to B-7, C-1 to C-7 & P"):
an adjuvant selected from the group of substances consisting of petroleum oils (alternative name) (628) + TX,
an insect control active substance selected from abamectin + TX, acequinocyl + TX, acetamiprid + TX, acetoprole + TX, acrinathrin + TX, acynonapyr + TX, afidopyropen + TX, afoxolaner + TX, alanycarb + TX, allethrin + TX, alpha-cypermethrin + TX, alphamethrin + TX, amidoflumet + TX, aminocarb + TX, azocyclotin + TX, bensultap + TX, benzoximate + TX, benzpyrimoxan + TX, betacyfluthrin + TX, beta-cypermethrin + TX, bifenazate + TX, bifenthrin + TX, binapacryl + TX, bioallethrin + TX, S-bioallethrin + TX, bioresmethrin + TX, bistrifluron + TX, broflanilide + TX, brofluthrinate + TX, bromophos-ethyl + TX, buprofezine + TX, butocarboxim + TX, cadusafos + TX, carbaryl + TX, carbosulfan + TX, cartap + TX, CAS number: 1632218-00-8 + TX, CAS number: 1808115-49-2 + TX, CAS number: 2032403-97-5 + TX, CAS number: 2044701-44-0 + TX, CAS number: 2128706-05-6 + TX, CAS number: 2095470-94-1 + TX, CAS number: 2377084-09-6 + TX, CAS number: 1445683-71-5 + TX, CAS number: 2408220-94-8 + TX, CAS number: 2408220-91-5 + TX, CAS number: 1365070-72-9 + TX, CAS number: 2171099-09-3 + TX, CAS number: 2396747-83-2 + TX, CAS number: 2133042-31-4 + TX, CAS number: 2133042-44-9 + TX, CAS number: 1445684-82-1 + TX, CAS number: 1445684-82-1 + TX, CAS number: 1922957-45-6 + TX, CAS number: 1922957-46-7 + TX, CAS number: 1922957-47-8 + TX, CAS number: 1922957-48-9 + TX, CAS number: 2415706-16-8 + TX, CAS number: 1594624-87-9 + TX, CAS number: 1594637-65-6 + TX, CAS number: 1594626-19-3 + TX, CAS number: 1990457-52-7 + TX, CAS number: 1990457-55-0 + TX, CAS number: 1990457-57-2 + TX, CAS number: 1990457-77-6 + TX, CAS number: 1990457-66-3 + TX, CAS number: 1990457-85-6 + TX, CAS number: 2220132-55-6 + TX, CAS number: 1255091-74-7 + TX, CAS number: 1305319-70-3 + TX, CAS number: 1442448-92-1 + TX, chlorantraniliprole + TX, chlordane + TX, chlorfenapyr + TX, chloroprallethrin + TX, chromafenozide + TX, clenpirin + TX, cloethocarb + TX, clothianidin + TX, 2-chlorophenyl N-methylcarbamate (CPMC) + TX, cyanofenphos + TX, cyantraniliprole + TX, cyclaniliprole + TX, cyclobutrifluram + TX, cycloprothrin + TX, cycloxaprid + TX, cyenopyrafen + TX, cyetpyrafen (or etpyrafen) + TX, cyflumetofen + TX, cyfluthrin + TX, cyhalodiamide + TX, cyhalothrin + TX, cypermethrin + TX, cyphenothrin + TX, cyproflanilide + TX, cyromazine + TX, deltamethrin + TX, diafenthiuron + TX, dialifos + TX, dibrom + TX, dicloromezotiaz + TX, diflovidazine + TX, diflubenzuron + TX, dimpropyridaz + TX, dinactin + TX, dinocap + TX, dinotefuran + TX, dioxabenzofos + TX, emamectin (or emamectin benzoate) + TX, empenthrin + TX, epsilon - momfluorothrin + TX, epsilon-metofluthrin + TX, esfenvalerate + TX, ethion + TX, ethiprole + TX, etofenprox + TX, etoxazole + TX, famphur + TX, fenazaquin + TX, fenfluthrin + TX, , fenmezoditiaz + TX, fenitrothion + TX, fenobucarb + TX, fenothiocarb + TX, fenoxycarb + TX, fenpropathrin + TX, fenpyroximate + TX, fensulfothion + TX, fenthion + TX, fentinacetate + TX, fenvalerate + TX, fipronil + TX, flometoquin + TX, flonicamid + TX, fluacrypyrim + TX, fluazaindolizine + TX, fluazuron + TX, flubendiamide + TX, flubenzimine + TX, fluchlordiniliprole + TX, flucitrinate + TX, flucycloxuron + TX, flucythrinate + TX, fluensulfone + TX, flufenerim + TX, flufenprox + TX, flufiprole + TX, fluhexafon + TX, flumethrin + TX, fluopyram + TX, flupentiofenox + TX, flupyradifurone + TX, flupyrimin + TX, fluralaner + TX, fluvalinate + TX, fluxametamide + TX, fosthiazate + TX, gamma-cyhalothrin + TX, Gossyplure^{™} + TX, guadipyr + TX, halofenozide + TX, halfenprox + TX, heptafluthrin + TX, hexythiazox + TX, hydramethylnon + TX, imicyafos + TX, imidacloprid + TX, imiprothrin + TX, indazapyroxamet + TX, indoxacarb + TX, iodomethane + TX, iprodione + TX, isocycloseram + TX, isothioate + TX, ivermectin + TX, kappa-bifenthrin + TX, kappa-tefluthrin + TX, lambda-Cyhalothrin + TX, lepimectin + TX, lotilaner + TX, lufenuron + TX, metaflumizone + TX, metaldehyde + TX, metam + TX, methomyl + TX, methoxyfenozide + TX, metofluthrin + TX, metolcarb + TX, mexacarbate + TX, milbemectin + TX, momfluorothrin + TX, niclosamide + TX, nicofluprole + TX; nitenpyram + TX, nithiazine + TX, omethoate + TX, oxamyl + TX, oxazosulfyl + TX, parathion-ethyl + TX, permethrin + TX, phenothrin + TX, phosphocarb + TX, piperonylbutoxide + TX, pirimicarb + TX, pirimiphos-ethyl + TX, pirimiphos-methyl + TX, Polyhedrosis virus + TX, prallethrin + TX, profenofos + TX, profluthrin + TX, propargite + TX, propetamphos + TX, propoxur + TX, prothiophos + TX, protrifenbute + TX, pyflubumide + TX, pymetrozine + TX, pyraclofos + TX, pyrafluprole + TX, pyridaben + TX, pyridalyl + TX, pyrifluquinazon + TX, pyrimidifen + TX, pyriminostrobin + TX, pyriprole + TX, pyriproxyfen + TX, resmethrin + TX, sarolaner + TX, selamectin + TX, silafluofen + TX, spinetoram + TX, spinosad + TX, spirodiclofen + TX, spiromesifen + TX, spiropidion + TX, spirotetramat + TX, spidoxamat + TX, sulfoxaflor + TX, tebufenozide + TX, tebufenpyrad + TX, tebupirimiphos + TX, tefluthrin + TX, temephos + TX, tetrachlorantraniliprole + TX, tetradiphon + TX, tetramethrin + TX, tetramethylfluthrin + TX, tetranactin + TX, tetraniliprole + TX, theta-cypermethrin + TX, thiacloprid + TX, thiamethoxam + TX, thiocyclam + TX, thiodicarb + TX, thiofanox + TX, thiometon + TX, thiosultap + TX, tigolaner + TX, tioxazafen + TX, tolfenpyrad + TX, toxaphene + TX, tralomethrin + TX, transfluthrin + TX, triazamate + TX, triazophos + TX, trichlorfon + TX, trichloronate + TX, trichlorphon + TX, trifluenfuronate + TX, triflumezopyrim + TX, tyclopyrazoflor + TX, zeta-cypermethrin + TX, Extract of seaweed and fermentation product derived from melasse + TX, Extract of seaweed and fermentation product derived from melasse comprising urea + TX, amino acids + TX, potassium and molybdenum and EDTA-chelated manganese + TX, Extract of seaweed and fermented plant products + TX, Extract of seaweed and fermented plant products comprising phytohormones + TX, vitamins + TX, EDTA-chelated copper + TX, zinc + TX, and iron + TX, azadirachtin + TX, Bacillus aizawai + TX, Bacillus chitinosporus AQ746 (NRRL Accession No B-21 618) + TX, Bacillus firmus + TX, Bacillus kurstaki + TX, Bacillus mycoides AQ726 (NRRL Accession No. B-21664) + TX, Bacillus pumilus (NRRL Accession No B-30087) + TX, Bacillus pumilus AQ717 (NRRL Accession No. B-21662) + TX, Bacillus sp. AQ178 (ATCC Accession No. 53522) + TX, Bacillus sp. AQ175 (ATCC Accession No. 55608) + TX, Bacillus sp. AQ177 (ATCC Accession No. 55609) + TX, Bacillus subtilis unspecified + TX, Bacillus subtilis AQ153 (ATCC Accession No. 55614) + TX, Bacillus subtilis AQ30002 (NRRL Accession No. B-50421) + TX, Bacillus subtilis AQ30004 (NRRL Accession No. B- 50455) + TX, Bacillus subtilis AQ713 (NRRL Accession No. B-21661) + TX, Bacillus subtilis AQ743 (NRRL Accession No. B-21665) + TX, Bacillus thuringiensis AQ52 (NRRL Accession No. B-21619) + TX, Bacillus thuringiensis BD#32 (NRRL Accession No B-21530) + TX, Bacillus thuringiensis subspec. kurstaki BMP 123 + TX, Beauveria bassiana + TX, D-limonene + TX, Granulovirus + TX, Harpin + TX, Helicoverpa armigera Nucleopolyhedrovirus + TX, Helicoverpa zea Nucleopolyhedrovirus + TX, Heliothis virescens Nucleopolyhedrovirus + TX, Heliothis punctigera Nucleopolyhedrovirus + TX, Metarhizium spp. + TX, Muscodor albus 620 (NRRL Accession No. 30547) + TX, Muscodor roseus A3-5 (NRRL Accession No. 30548) + TX, Neem tree based products + TX, Paecilomyces fumosoroseus + TX, Paecilomyces lilacinus + TX, Pasteuria nishizawae + TX, Pasteuria penetrans + TX, Pasteuria ramosa + TX, Pasteuria thornei + TX, Pasteuria usgae + TX, P-cymene + TX, Plutella xylostella Granulosis virus + TX, Plutella xylostella Nucleopolyhedrovirus + TX, Polyhedrosis virus + TX, pyrethrum + TX, QRD 420 (a terpenoid blend) + TX, QRD 452 (a terpenoid blend) + TX, QRD 460 (a terpenoid blend) + TX, Quillaja saponaria + TX, Rhodococcus globerulus AQ719 (NRRL Accession No B-21663) + TX, Spodoptera frugiperda Nucleopolyhedrovirus + TX, Streptomyces galbus (NRRL Accession No. 30232) + TX, Streptomyces sp. (NRRL Accession No. B-30145) + TX, Terpenoid blend + TX, and Verticillium spp. + TX;
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX;
an anthelmintic selected from the group of substances consisting of abamectin (1) + TX, crufomate (1011) + TX, cyclobutrifluram + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX;
an avicide selected from the group of substances consisting of chloralose (127) + TX, endrin (1122) + TX, fenthion (346) + TX, pyridin-4-amine (IUPAC name) (23) and strychnine (745) + TX;
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX;
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX;
a soil sterilant selected from the group of substances consisting of iodomethane (IUPAC name) (542) and methyl bromide (537) + TX;
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX;
an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (*E,Z*)-tetradeca-4,10-dien-1-yl acetate (IUPAC name) (779) + TX, (*Z*)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (*Z*)-hexadec-13-en-11-yn-1-yl acetate (IUPAC name) (438) + TX, (Z)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (Z)-tetradec-9-en-1-yl acetate (IUPAC name) (784) + TX, (7*E*,9*Z*)-dodeca-7,9-dien-1-yl acetate (IUPAC name) (283) + TX, (9*Z*,11*E*)-tetradeca-9,11-dien-1-yl acetate (IUPAC name) (780) + TX, (*9*Z,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX;
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX;
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX;
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cyclobutrifluram + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX;
a nitrification inhibitor selected from the group of substances consisting of potassium ethylxanthate [CCN] and nitrapyrin (580) + TX;
a plant activator selected from the group of substances consisting of acibenzolar (6) + TX, acibenzolar-S-methyl (6) + TX, probenazole (658) and *Reynoutria sachalinensis* extract (alternative name) (720) + TX;
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (including alpha-bromadiolone) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX;
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX;
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX;
a virucide selected from the group of substances consisting of imanin (alternative name) [CCN] and ribavirin (alternative name) [CCN] + TX;
a wound protectant selected from the group of substances consisting of mercuric oxide (512) + TX, octhilinone (590) and thiophanate-methyl (802) + TX;
a biologically active substance selected from 1,1-bis(4-chloro-phenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxa-fos + TX, benzyl benzoate + TX, bixafen + TX, brofenvalerate + TX, bromo-cyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chino-methionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dino-penton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fen-pyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methylpropyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX , Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8 + TX, 10-dien-1-yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)-ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)-ethane + TX, 1,2-dichloropropane with 1,3-dichloropropene + TX, 1-bromo-2-chloroethane + TX, 2,2,2-trichloro-1-(3,4-dichloro-phenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethyl-carbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, EI 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl O-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1,2-dibromo-3-chloropropane + TX, 1,3-dichloropropene + TX, 3,4-dichlorotetrahydrothio-phene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, anisiflupurin + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX ,acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, -sodium fluoroacetate + TX, thallium sulfate + TX, warfarin + TX, -2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, chloroinconazide + TX, mercuric oxide + TX, thiophanate-methyl + TX, azaconazole + TX, bitertanol + TX, bromuconazole + TX, cyproconazole + TX, difenoconazole + TX, diniconazole -+ TX, epoxiconazole + TX, fenbuconazole + TX, fluquinconazole + TX, flusilazole + TX, flutriafol + TX, furametpyr + TX, hexaconazole + TX, imazalil- + TX, imiben-conazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, -simeconazole + TX, tebucon-azole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidin + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, -metalaxyl -+ TX, Rmetalaxyl + TX, ofurace + TX, oxadixyl + TX, carbendazim + TX, debacarb + TX, fuberidazole -+ TX, thiabendazole + TX, chlozolinate + TX, dichlozoline + TX, myclozoline- + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoximmethyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb -+ TX, chloro-tha-lonil + TX, cyflufenamid + TX, cymoxanil + TX, cyclobutrifluram + TX, diclocymet + TX, diclomezine -+ TX, dicloran + TX, diethofencarb + TX, dimethomorph -+ TX, flumorph + TX, dithianon + TX, ethaboxam + TX, etridiazole + TX, famoxadone + TX, fenamidone + TX, fenoxanil + TX, ferimzone + TX, fluazinam + TX, flumetylsulforim + TX, fluopicolide + TX, fluoxytioconazole + TX, flusulfamide + TX, fluxapyroxad + TX, -fenhexamid + TX, fosetyl-aluminium -+ TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluoro-phenyl)-2,5-dimethyl-pyrazol-3-amine + TX, 4- (2- bromo- 4- fluorophenyl) - N- (2- chloro- 6- fluorophenyl) - 1, 3- dimethyl- 1H- pyrazol-5- amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chlorophenyl] methanesulfonate + TX, but-3-ynyl N-[6-[[(Z)-[(1-methyltetrazol-5-yl)-phenylmethylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, metarylpicoxamid + TX, tebufloquin + TX, ipflufenoquin + TX, quinofumelin + TX, isofetamid + TX, N-[2-[2,4-dichloro-phenoxy]phenyl]-3-(difluoromethyl)-1-methyl-pyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flufenoxadiazam + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) - N- ((3R) - 1, 1, 3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2- (difluoromethyl) - N- ((3R) - 1, 1, 3- trimethylindan- 4- yl) pyridine- 3-carboxamide + TX, α- (1, 1- dimethylethyl) - α- [4'- (trifluoromethoxy) [1, 1'- biphenyl] - 4- yl] -5-pyrimidinemethanol + TX, fluoxapiprolin + TX, enoxastrobin + TX, 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, seboctylamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxy-ethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[(2-trifluoromethyl)oxetan-2-yl]phenyl]-N-methyl-formamidine + TX, N-ethyl-N'-[5-methoxy-2-methyl-4-[(2-trifuoromethyl)tetrahydrofuran-2-yl]phenyl]-N-methyl-formamidine + TX (these compounds may be prepared from the methods described in WO2019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-flluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380); 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX. The compounds in this paragraph may be prepared from the methods described in WO 2017/055473, WO 2017/055469, WO 2017/093348 and WO 2017/118689; 2-[6-(4-chlorophenoxy)-2-(trifluoromethyl)-3-pyridyl]-1-(1,2,4-triazol-1-yl)propan-2-ol + TX (this compound may be prepared from the methods described in WO 2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO 2017/029179); 3-[2-(1-chlorocyclopropyl)-3-(2-fluorophenyl)-2-hydroxy-propyl]imidazole-4-carbonitrile + TX (this compound may be prepared from the methods described in WO 2016/156290); 3-[2-(1-chlorocyclopropyl)-3-(3-chloro-2-fluoro-phenyl)-2-hydroxy-propyl]imidazole-4-carbonitrile + TX (this compound may be prepared from the methods described in WO 2016/156290); (4-phenoxyphenyl)methyl 2-amino-6-methyl-pyridine-3-carboxylate + TX (this compound may be prepared from the methods described in WO 2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO 2011/138281); N-methyl-4-[5-(trifluoromethyl)-1,2,4-oxadiazol-3-yl]benzenecarbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO 2018/153707); N'-(2-chloro-5-methyl-4-phenoxyphenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO 2016/202742); 2-(difluoromethyl)-N-[(3S)-3-ethyl-1,1-dimethyl-indan-4-yl]pyridine-3-carboxamide + TX (this compound may be prepared from the methods described in WO 2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO 2017/220485); 2-oxo-N-propyl-2-[4-[5-(trifluoromethyl)-1,2,4-oxadiazol-3-yl]phenyl]acetamide + TX (this compound may be prepared from the methods described in WO 2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO 2018/158365); 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO 2018/202428);
microbials including: *Acinetobacter Iwoffii* + TX, *Acremonium alternatum* + TX + TX, *Acremonium* cephalosporium + TX + TX, *Acremonium diospyri* + TX, *Acremonium obclavatum* + TX, *Adoxophyes orana granulovirus* (AdoxGV) (Capex^{®}) + TX, *Agrobacterium radiobacter* strain K84 (Galltrol-A^{®}) + TX, *Alternaria alternate* + TX, *Alternaria cassia* + TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans* + TX, *Azospirillum* + TX, (MicroAZ^{®} + TX, TAZO B^{®}) + TX, *Azotobacter* + TX, *Azotobacter chroocuccum* (Azotomeal^{®}) + TX, *Azotobacter* cysts (Bionatural Blooming Blossoms^{®}) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans* + TX, *Bacillus firmus* (BioSafe^{®} + TX, BioNem-WP^{®} + TX, VOTiVO^{®}) + TX, *Bacillus firmus* strain I-1582 + TX, *Bacillus macerans* + TX, *Bacillus marismortui* + TX, *Bacillus megaterium* + TX, *Bacillus mycoides* strain AQ726 + TX, *Bacillus papillae* (Milky Spore Powder^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ^{®}) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* Cry1Ab + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPel^{®}) + TX, bacteria spp. (GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis* + TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii* + TX, *Burkholderia gladioli* + TX, *Burkholderia* spp. + TX, Canadian thistle fungus (CBH Canadian Bioherbicide^{®}) + TX, *Candida butyri* + TX, *Candida famata* + TX, *Candida fructus* + TX, *Candida glabrata* + TX, *Candida guilliermondii* + TX, *Candida melibiosica* + TX, *Candida oleophila* strain O + TX, *Candida parapsilosis* + TX, *Candida pelliculosa* + TX, *Candida pulcherrima* + TX, *Candida reukaufii* + TX, *Candida saitoana* (Bio-Coat^{®} + TX, Biocure^{®}) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides* + TX, *Cladosporium oxysporum* + TX, *Cladosporium chlorocephalum* + TX, *Cladosporium* spp. + TX, *Cladosporium tenuissimum* + TX, *Clonostachys rosea* (EndoFine^{®}) + TX, *Colletotrichum acutatum* + TX, *Coniothyrium minitans* (Cotans WG^{®}) + TX, *Coniothyrium* spp. + TX, *Cryptococcus albidus* (YIELDPLUS^{®}) + TX, *Cryptococcus humicola* + TX, *Cryptococcus infirmominiatus* + TX, *Cryptococcus laurentii* + TX, *Cryptophlebia leucotreta granulovirus* (Cryptex^{®}) + TX, *Cupriavidus campinensis* + TX, *Cydia pomonella granulovirus* (CYD-X^{®}) + TX, *Cydia pomonella granulovirus* (Madex^{®} + TX, Madex Plus^{®} + TX, Madex Max/ Carpovirusine^{®}) + TX, *Cylindrobasidium laeve* (Stumpout^{®}) + TX, *Cylindrocladium* + TX, *Debaryomyces hansenii* + TX, *Drechslera hawaiinensis* + TX, *Enterobacter cloacae* + TX, *Enterobacteriaceae* + TX, *Entomophtora virulenta* (Vektor^{®}) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean^{®} / Biofox C^{®}) + TX, *Fusarium proliferatum* + TX, *Fusarium* spp. + TX, *Galactomyces geotrichum* + TX, *Gliocladium catenulatum* (Primastop^{®} + TX, Prestop^{®}) + TX, *Gliocladium roseum* + TX, *Gliocladium* spp. (SoilGard^{®}) + TX, *Gliocladium virens* (Soilgard^{®}) + TX, *Granulovirus* (Granupom^{®}) + TX, *Halobacillus halophilus* + TX, *Halobacillus litoralis* + TX, *Halobacillus trueperi* + TX, *Halomonas* spp. + TX, *Halomonas subglaciescola* + TX, *Halovibrio variabilis* + TX, *Hanseniaspora uvarum* + TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata* + TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus* + TX, *Meira geulakonigii* + TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima* + TX, *Microdochium dimerum* (Antibot^{®}) + TX, *Micromonospora coerulea* + TX, *Microsphaeropsis ochracea* + TX, *Muscodor albus* 620 (Muscudor^{®}) + TX, *Muscodor roseus* strain A3-5 + TX, *Mycorrhizae* spp. (AMykor^{®} + TX, Root Maximizer^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus* + TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa* + TX, *Pantoea agglomerans* (BlightBan C9-1^{®}) + TX, *Pantoea* spp. + TX, *Pasteuria* spp. (Econem^{®}) + TX, *Pasteuria nishizawae* + TX, *Penicillium aurantiogriseum* + TX, *Penicillium billai* (Jumpstart^{®} + TX, TagTeam^{®}) + TX, *Penicillium brevicompactum* + TX, *Penicillium frequentans* + TX, *Penicillium griseofulvum* + TX, *Penicillium purpurogenum* + TX, *Penicillium* spp. + TX, *Penicillium viridicatum* + TX, *Phlebiopsis gigantean* (Rotstop^{®}) + TX, phosphate solubilizing bacteria (Phosphomeal^{®}) + TX, *Phytophthora cryptogea* + TX, *Phytophthora palmivora* (Devine^{®}) + TX, *Pichia anomala* + TX, *Pichia guilermondii* + TX, *Pichia membranaefaciens* + TX, *Pichia onychis* + TX, *Pichia stipites* + TX, *Pseudomonas aeruginosa* + TX, *Pseudomonas aureofasciens* (Spot-Less Biofungicide^{®}) + TX, *Pseudomonas cepacia* + TX, *Pseudomonas chlororaphis* (AtEze^{®}) + TX, *Pseudomonas corrugate* + TX, *Pseudomonas fluorescens* strain A506 (BlightBan A506^{®}) + TX, *Pseudomonas putida* + TX, *Pseudomonas reactans* + TX, *Pseudomonas* spp. + TX, *Pseudomonas syringae* (Bio-Save^{®}) + TX, *Pseudomonas viridiflava* + TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum* + TX, *Pythium oligandrum* (Polygandron^{®} + TX, Polyversum^{®}) + TX, *Pythium periplocum* + TX, *Rhanella aquatilis* + TX, *Rhanella* spp. + TX, *Rhizobia* (Dormal^{®} + TX, Vault^{®}) + TX, *Rhizoctonia* + TX, *Rhodococcus globerulus* strain AQ719 + TX, *Rhodosporidium diobovatum* + TX, *Rhodosporidium toruloides* + TX, *Rhodotorula* spp. + TX, *Rhodotorula glutinis* + TX, *Rhodotorula graminis* + TX, *Rhodotorula mucilagnosa* + TX, *Rhodotorula rubra* + TX, *Saccharomyces cerevisiae* + TX, *Salinococcus roseus* + TX, *Sclerotinia minor* + TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens* + TX, *Serratia plymuthica* + TX, *Serratia* spp. + TX, *Sordaria fimicola* + TX, *Spodoptera littoralis nucleopolyhedrovirus* (Littovir^{®}) + TX, *Sporobolomyces roseus* + TX, *Stenotrophomonas maltophilia* + TX, *Streptomyces ahygroscopicus* + TX, *Streptomyces albaduncus* + TX, *Streptomyces exfoliates* + TX, *Streptomyces galbus* + TX, *Streptomyces griseoplanus* + TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus* + TX, *Tilletiopsis minor* + TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycostar^{®}) + TX, *Trichoderma harzianum* T-22 (Trianum-P^{®} + TX, PlantShield HC^{®} + TX, RootShield^{®} + TX, Trianum-G^{®}) + TX, *Trichoderma harzianum* T-39 (Trichodex^{®}) + TX, *Trichoderma inhamatum* + TX, *Trichoderma koningii* + TX, *Trichoderma* spp. LC 52 (Sentinel^{®}) + TX, *Trichoderma lignorum* + TX, *Trichoderma longibrachiatum* + TX, *Trichoderma polysporum* (Binab T^{®}) + TX, *Trichoderma taxi* + TX, *Trichoderma virens* + TX, *Trichoderma virens* (formerly Gliocladium virens GL-21) (SoilGuard^{®}) + TX, *Trichoderma viride* + TX, *Trichoderma viride* strain ICC 080 (Remedier^{®}) + TX, *Trichosporon pullulans* + TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen^{®}) + TX, *Ustilago maydis* + TX, various bacteria and supplementary micronutrients (Natural ∥^{®}) + TX, various fungi (Millennium Microbes^{®}) + TX, *Verticillium chlamydosporium* + TX, *Verticillium lecanii* (Mycotal^{®} + TX, Vertalec^{®}) + TX, Vip3Aa20 (VIPtera^{®}) + TX, *Virgibaclillus marismortui* + TX, *Xanthomonas campestris pv. Poae* (Camperico^{®}) + TX, *Xenorhabdus bovienii* + TX, *Xenorhabdus nematophilus*;
Plant extracts including: pine oil (Retenol^{®}) + TX, azadirachtin (Plasma Neem Oil^{®} + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina* + TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNlQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®});
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromone^{®}) + TX, Peachtree Borer Pheromone (Isomate-P^{®}) + TX, Tomato Pinworm Pheromone (3M Sprayable pheromone^{®}) + TX, Entostat powder (extract from palm tree) (Exosex CM^{®}) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate; Macrobials including: *Aphelinus abdominalis* + TX, *Aphidius ervi* (Aphelinus-System^{®}) + TX, *Acerophagus papaya* + TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola* + TX, *Ageniaspis fuscicollis* + TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum* + TX, *Anagrus atomus* + TX, *Anagyrus fusciventris* + TX, *Anagyrus kamali* + TX, *Anagyrus loecki* + TX, *Anagyrus pseudococci* (Citripar^{®}) + TX, *Anicetus benefices* + TX, *Anisopteromalus calandrae* + TX, *Anthocoris nemoralis* (Anthocoris-System^{®}) + TX, *Aphelinus abdominalis* (Apheline^{®} + TX, Aphiline^{®}) + TX, *Aphelinus asychis* + TX, *Aphidius colemani* (Aphipar^{®}) + TX, *Aphidius ervi* (Ervipar^{®}) + TX, *Aphidius gifuensis* + TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis* + TX, *Aphytis melinus* + TX, *Aprostocetus hagenowii* + TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis* + TX, *Chilocorus nigritus* + TX, *Chrysoperla carnea* (Chrysoline^{®}) + TX, *Chrysoperla carnea* (Chrysopa^{®}) + TX, *Chrysoperla rufilabris* + TX, *Cirrospilus ingenuus* + TX, *Cirrospilus quadristriatus* + TX, *Citrostichus phyllocnistoides* + TX, *Closterocerus chamaeleon* + TX, *Closterocerus* spp. + TX, *Coccidoxenoides perminutus* (Planopar^{®}) + TX, *Coccophagus cowperi* + TX, *Coccophagus lycimnia* + TX, *Cotesia flavipes* + TX, *Cotesia plutellae* + TX, *Cryptolaemus montrouzieri* (Cryptobug^{®} + TX, Cryptoline^{®}) + TX, *Cybocephalus nipponicus* + TX, *Dacnusa sibirica* + TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus* + TX, *Diachasmimorpha krausii* + TX, *Diachasmimorpha longicaudata* + TX, *Diaparsis jucunda* + TX, *Diaphorencyrtus aligarhensis* + TX, *Diglyphus isaea* + TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae* + TX, *Encarsia haitiensis* + TX, *Episyrphus balteatus* (Syrphidend^{®}) + TX, *Eretmoceris siphonini* + TX, *Eretmocerus californicus* + TX, *Eretmocerus eremicus* (Ercal^{®} + TX, Eretline e^{®}) + TX, *Eretmocerus eremicus* (Bemimix^{®}) + TX, *Eretmocerus hayati* + TX, *Eretmocerus mundus* (Bemipar^{®} + TX, Eretline m^{®}) + TX, *Eretmocerus siphonini* + TX, *Exochomus quadripustulatus* + TX, *Feltiella acarisuga* (Spidend^{®}) + TX, *Feltiella acarisuga* (Feltiline^{®}) + TX, *Fopius arisanus* + TX, *Fopius ceratitivorus* + TX, Formononetin (Wirless Beehome^{®}) + TX, *Franklinothrips vespiformis* (Vespop^{®}) + TX, *Galendromus occidentalis* + TX, *Goniozus legneri* + TX, *Habrobracon hebetor* + TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens* + TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides* + TX, *Lecanoideus floccissimus* + TX, *Lemophagus errabundus* + TX, *Leptomastidea abnormis* + TX, *Leptomastix dactylopii* (Leptopar^{®}) + TX, *Leptomastix epona* + TX, *Lindorus lophanthae* + TX, *Lipolexis oregmae* + TX, *Lucilia caesar* (Natuflly^{®}) + TX, *Lysiphlebus testaceipes* + TX, *Macrolophus caliginosus* (Mirical-N^{®} + TX, Macroline c^{®} + TX, Mirical^{®}) + TX, *Mesoseiulus longipes* + TX, *Metaphycus flavus* + TX, *Metaphycus lounsburyi* + TX, *Micromus angulatus* (Milacewing^{®}) + TX, *Microterys flavus* + TX, *Muscidifurax raptorellus* and *Spalangia cameroni* (Biopar^{®}) + TX, *Neodryinus typhlocybae* + TX, *Neoseiulus californicus* + TX, *Neoseiulus cucumeris* (THRYPEX^{®}) + TX, *Neoseiulus fallacis* + TX, *Nesideocoris tenuis* (NesidioBug^{®} + TX, Nesibug^{®}) + TX, *Ophyra aenescens* (Bioflly^{®}) + TX, *Orius insidiosus* (Thripor-I^{®} + TX, Oriline i^{®}) + TX, *Orius laevigatus* (Thripor-L^{®} + TX, Oriline I^{®}) + TX, *Orius majusculus* (Oriline m^{®}) + TX, *Orius strigicollis* (Thripor-S^{®}) + TX, *Pauesia juniperorum* + TX, *Pediobius foveolatus* + TX, *Phasmarhabditis hermaphrodita* (Nemaslug^{®}) + TX, *Phymastichus coffea* + TX, *Phytoseiulus macropilus* + TX, *Phytoseiulus persimilis* (Spidex^{®} + TX, Phytoline p^{®}) + TX, *Podisus maculiventris* (Podisus^{®}) + TX, *Pseudacteon curvatus* + TX, *Pseudacteon obtusus* + TX, *Pseudacteon tricuspis* + TX, *Pseudaphycus maculipennis* + TX, *Pseudleptomastix mexicana* + TX, *Psyllaephagus pilosus* + TX, *Psyttalia concolor* (complex) + TX, *Quadrastichus* spp. + TX, *Rhyzobius lophanthae* + TX, *Rodolia cardinalis* + TX, *Rumina decollate* + TX, *Semielacher petiolatus* + TX, *Sitobion avenae* (Ervibank^{®}) + TX, *Steinernema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinernema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline sf^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinernema* spp. + TX, *Steinernematid* spp. (Guardian Nematodes^{®}) + TX, *Stethorus punctillum* (Stethorus^{®}) + TX, *Tamarixia radiate* + TX, *Tetrastichus setifer* + TX, *Thripobius semiluteus* + TX, *Torymus sinensis* + TX, *Trichogramma brassicae* (Tricholine b^{®}) + TX, *Trichogramma brassicae* (Tricho-Strip^{®}) + TX, *Trichogramma evanescens* + TX, *Trichogramma minutum* + TX, *Trichogramma ostriniae* + TX, *Trichogramma platneri* + TX, *Trichogramma pretiosum* + TX, *Xanthopimpla stemmator;* other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae* + TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo^{®}) + TX and Traps (Takitrapline y + b^{®}) + TX;

(1) **antibacterial agents** selected from the group of:
   (1.1) bacteria, examples of which are *Bacillus mojavensis* strain R3B (Accession No. NCAIM (P) B001389) (WO 2013/034938) from Certis USA LLC, a subsidiary of Mitsui & Co. + TX; *Bacillus pumilus,* in particular strain BU F-33, having NRRL Accession No. 50185 (available as part of the CARTISSA^{®} product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661, U.S. Patent No. 6,060,051) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus subtilis* CX-9060 from Certis USA LLC, a subsidiary of Mitsui & Co. + TX; *Bacillus* sp., in particular strain D747 (available as DOUBLE NICKEL^{®} from Kumiai Chemical Industry Co., Ltd.), having Accession No. FERM BP-8234, U.S. Patent No. 7,094,592 + TX; *Paenibacillus* sp. strain having Accession No. NRRL B-50972 or Accession No. NRRL B-67129, WO 2016/154297 + TX; *Paenibacillus polymyxa,* in particular strain AC-1 (e.g. TOPSEED^{®} from Green Biotech Company Ltd.) + TX; *Pantoea agglomerans,* in particular strain E325 (Accession No. NRRL B-21856) (available as BLOOMTIME BIOLOGICAL^{™} FD BIOPESTICIDE from Northwest Agri Products) + TX; *Pseudomonas proradix* (e.g. PRORADIX^{®} from Sourcon Padena) + TX; and
   (1.2) fungi, examples of which are *Aureobasidium pullulans,* in particular blastospores of strain DSM14940, blastospores of strain DSM 14941 or mixtures of blastospores of strains DSM14940 and DSM14941 (e.g., BOTECTOR^{®} and BLOSSOM PROTECT^{®} from bio-ferm, CH) + TX; *Pseudozyma* *aphidis* (as disclosed in WO2011/151819 by Yissum Research Development Company of the Hebrew University of Jerusalem) + TX; Saccharomyces cerevisiae, in particular strains CNCM No. 1-3936, CNCM No. 1-3937, CNCM No. 1-3938 or CNCM No. 1-3939 (WO 2010/086790) from Lesaffre et Compagnie, FR;
(2) **biological fungicides** selected from the group of:
   (2.1) bacteria, examples of which are *Agrobacterium radiobacter* strain K84 (e.g. GALLTROL-A^{®} from AgBioChem, CA) + TX; *Agrobacterium radiobacter* strain K1026 (e.g. NOGALL^{™} from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus amyloliquefaciens,* in particular strain D747 (available as Double Nickel^{™} from Kumiai Chemical Industry Co., Ltd., having accession number FERM BP-8234, US Patent No. 7,094,592) + TX; *Bacillus amyloliquefaciens* strain F727 (also known as strain MBI110) (NRRL Accession No. B-50768, WO 2014/028521) (STARGUS^{®} from Marrone Bio Innovations) + TX; *Bacillus amyloliquefaciens* strain FZB42, Accession No. DSM 23117 (available as RHIZOVITAL^{®} from ABiTEP, DE) + TX; *Bacillus amyloliquefaciens* isolate B246 (e.g. AVOGREEN^{™} from University of Pretoria) + TX; *Bacillus licheniformis,* in particular strain SB3086, having Accession No. ATCC 55406, WO 2003/000051 (available as ECOGUARD^{®} Biofungicide and GREEN RELEAF^{™} from Novozymes) + TX + TX; *Bacillus licheniformis* FMCH001 and *Bacillus subtilis* FMCH002 (QUARTZO^{®} (WG) and PRESENCE^{®} (WP) from FMC Corporation) + TX; *Bacillus methylotrophicus* strain BAC-9912 (from Chinese Academy of Sciences' Institute of Applied Ecology) + TX; *Bacillus mojavensis* strain R3B (Accession No. NCAIM (P) B001389) (WO 2013/034938) from Certis USA LLC, a subsidiary of Mitsui & Co. + TX; *Bacillus mycoides,* isolate, having Accession No. B-30890 (available as BMJ TGAI^{®} or WG and LifeGard^{™} from Certis USA LLC, a subsidiary of Mitsui & Co.) + TX; *Bacillus pumilus,* in particular strain QST2808 (available as SONATA^{®} from Bayer CropScience LP, US, having Accession No. NRRL B-30087 and described in U.S. Patent No. 6,245,551) + TX; *Bacillus pumilus,* in particular strain GB34 (available as Yield Shield^{®} from Bayer AG, DE) + TX; *Bacillus pumilus,* in particular strain BU F-33, having NRRL Accession No. 50185 (available as part of the CARTISSA product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661 and described in U.S. Patent No. 6,060,051) + TX; *Bacillus subtilis* Y1336 (available as BIOBAC^{®} WP from Bion-Tech, Taiwan, registered as a biological fungicide in Taiwan under Registration Nos. 4764, 5454, 5096 and 5277) + TX; *Bacillus subtilis* strain MBI 600 (available as SUBTILEX from BASF SE), having Accession Number NRRL B-50595, U.S. Patent No. 5,061,495 + TX; *Bacillus subtilis* strain GB03 (available as Kodiak^{®} from Bayer AG, DE) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* CX-9060 from Certis USA LLC, a subsidiary of Mitsui & Co. + TX; *Bacillus subtilis* KTSB strain (FOLIACTIVE^{®} from Donaghys) + TX; *Bacillus subtilis* IAB/BS03 (AVIV^{™} from STK Bio-Ag Technologies, PORTENTO^{®} from Idai Nature) + TX; *Bacillus subtilis* strain Y1336 (available as BIOBAC^{®} WP from Bion-Tech, Taiwan, registered as a biological fungicide in Taiwan under Registration Nos. 4764, 5454, 5096 and 5277) + TX; *Paenibacillus epiphyticus* (WO 2016/020371) from BASF SE + TX; *Paenibacillus polymyxa* ssp. plantarum (WO 2016/020371) from BASF SE + TX; *Paenibacillus* sp. strain having Accession No. NRRL B-50972 or Accession No. NRRL B-67129, WO 2016/154297 + TX; *Pseudomonas chlororaphis* strain AFS009, having Accession No. NRRL B-50897, WO 2017/019448 (e.g., HOWLER^{™} and ZIO^{®} from AgBiome Innovations, US) + TX; *Pseudomonas chlororaphis,* in particular strain MA342 (e.g. CEDOMON^{®}, CERALL^{®}, and CEDRESS^{®} by Bioagri and Koppert) + TX; *Pseudomonas fluorescens* strain A506 (e.g. BLIGHTBAN^{®} A506 by NuFarm) + TX; *Pseudomonas proradix* (e.g. PRORADIX^{®} from Sourcon Padena) + TX; *Streptomyces griseoviridis* strain K61 (also known as *Streptomyces galbus* strain K61) (Accession No. DSM 7206) (MYCOSTOP^{®} from Verdera, PREFENCE^{®} from BioWorks, cf. Crop Protection 2006, 25, 468-475) + TX; *Streptomyces lydicus* strain WYEC108 (also known as *Streptomyces lydicus* strain WYCD108US) (ACTINO-IRON^{®} and ACTINOVATE^{®} from Novozymes) + TX; and
   (2.2) fungi, examples of which are *Ampelomyces quisqualis,* in particular strain AQ 10 (e.g. AQ 10^{®} by IntrachemBio Italia) + TX; *Ampelomyces quisqualis* strain AQ10, having Accession No. CNCM 1-807 (e.g., AQ 10^{®} by IntrachemBio Italia) + TX; *Aspergillus flavus* strain NRRL 21882 (products known as AFLA-GUARD^{®} from Syngenta/ChemChina) + TX; *Aureobasidium pullulans,* in particular blastospores of strain DSM14940 + TX; *Aureobasidium pullulans,* in particular blastospores of strain DSM 14941 + TX; *Aureobasidium pullulans,* in particular mixtures of blastospores of strains DSM14940 and DSM 14941 (e.g. Botector^{®} by bio-ferm, CH) + TX; *Chaetomium cupreum* (Accession No. CABI 353812) (e.g. BlOKUPRUM^{™} by AgriLife) + TX; *Chaetomium globosum* (available as RIVADIOM^{®} by Rivale) + TX; *Cladosporium cladosporioides,* strain H39, having Accession No. CBS122244, US 2010/0291039 (by Stichting Dienst Landbouwkundig Onderzoek) + TX; *Coniothyrium minitans,* in particular strain CON/M/91-8 (Accession No. DSM9660, e.g. Contans ^{®} from Bayer CropScience Biologics GmbH) + TX; *Cryptococcus flavescens,* strain 3C (NRRL Y-50378), (B2.2.99) + TX; *Dactylaria candida* + TX; Dilophosphora alopecuri (available as TWIST FUNGUS^{®}) + TX; Fusarium oxysporum, strain Fo47 (available as FUSACLEAN^{®} by Natural Plant Protection) + TX; Gliocladium catenulatum (Synonym: Clonostachys rosea f. catenulate) strain J1446 (e.g. Prestop ^{®} by Lallemand) + TX; Gliocladium roseum (also known as Clonostachys rosea f rosea), in particular strain 321U from Adjuvants Plus, strain ACM941 as disclosed in Xue (Efficacy of Clonostachys rosea strain ACM941 and fungicide seed treatments for controlling the root tot complex of field pea, Can Jour Plant Sci 83(3): 519-524), or strain IK726 (Jensen DF, et al. Development of a biocontrol agent for plant disease control with special emphasis on the near commercial fungal antagonist Clonostachys rosea strain 'IK726', Australas Plant Pathol. 2007,36:95-101) + TX; Lecanicillium lecanii (formerly known as Verticillium lecanii) conidia of strain KV01 (e.g. Vertalec^{®} by Koppert/Arysta) + TX; Metschnikowia fructicola, in particular strain NRRL Y-30752, (B2.2.3) + TX; Microsphaeropsis ochracea + TX; Muscodor roseus, in particular strain A3-5 (Accession No. NRRL 30548) + TX; Penicillium steckii (DSM 27859, WO 2015/067800) from BASF SE + TX; Penicillium vermiculatum + TX; Phlebiopsis gigantea strain VRA 1992 (ROTSTOP^{®} C from Danstar Ferment) + TX; Pichia anomala, strain WRL-076 (NRRL Y-30842), U.S. Patent No. 7,579,183 + TX; Pseudozyma flocculosa, strain PF-A22 UL (available as SPORODEX^{®} L by Plant Products Co., CA) + TX; Saccharomyces cerevisiae, in particular strain LASO2 (from Agro-Levures et Dérivés), strain LAS117 cell walls (CEREVISANE^{®} from Lesaffre, ROMEO^{®} from BASF SE), strains CNCM No. 1-3936, CNCM No. 1-3937, CNCM No. 1-3938, CNCM No. 1-3939 (WO 2010/086790) from Lesaffre et Compagnie, FR + TX; Simplicillium lanosoniveum + TX; Talaromyces flavus, strain V117b + TX; Trichoderma asperelloides JM41R (Accession No. NRRL B-50759) (TRICHO PLUS^{®} from BASF SE) + TX; Trichoderma asperellum, in particular, strain kd (e.g. T-Gro from Andermatt Biocontrol) + TX; Trichoderma asperellum, in particular strain SKT-1, having Accession No. FERM P-16510 (e.g. ECO-HOPE^{®} from Kumiai Chemical Industry), strain T34 (e.g. T34 Biocontrol by Biocontrol Technologies S.L., ES) or strain ICC 012 from Isagro + TX; Trichoderma atroviride, in particular strain SC1 (having Accession No. CBS 122089, WO 2009/116106 and U.S. Patent No. 8,431,120 (from Bi-PA)), strain 77B (T77 from Andermatt Biocontrol) or strain LU132 (e.g. Sentinel from Agrimm Technologies Limited) + TX; Trichoderma atroviride, strain CNCM 1-1237 (e.g. Esquive^{®} WP from Agrauxine, FR) + TX; Trichoderma atroviride, strain no. V08/002387 + TX; Trichoderma atroviride, strain NMI no. V08/002388 + TX; Trichoderma atroviride, strain NMI no. V08/002389 + TX; Trichoderma atroviride, strain NMI no. V08/002390 + TX; Trichoderma atroviride, strain LC52 (e.g. Tenet by Agrimm Technologies Limited) + TX; Trichoderma atroviride, strain ATCC 20476 (IMI 206040) + TX; Trichoderma atroviride, strain T11 (IMI352941/ CECT20498) + TX; Trichoderma atroviride, strain SKT-1 (FERM P-16510), JP Patent Publication (Kokai) 11-253151 A + TX; Trichoderma atroviride, strain SKT-2 (FERM P-16511), JP Patent Publication (Kokai) 11-253151 A + TX; Trichoderma atroviride, strain SKT-3 (FERM P-17021), JP Patent Publication (Kokai) 11-253151 A + TX; Trichoderma fertile (e.g. product TrichoPlus from BASF) + TX; Trichoderma gamsii (formerly T. viride), strain ICC080 (IMI CC 392151 CABI, e.g. BioDerma by AGROBIOSOL DE MEXICO, S.A. DE C.V.) + TX; Trichoderma gamsii (formerly T. viride), strain ICC 080 (IMI CC 392151 CABI) (available as BIODERMA^{®} by AGROBIOSOL DE MEXICO, S.A. DE C.V.) + TX; Trichoderma harmatum + TX; Trichoderma harmatum, having Accession No. ATCC 28012 + TX; Trichoderma harzianum strain T-22 (e.g. Trianum-P from Andermatt Biocontrol or Koppert) or strain Cepa SimbT5 (from Simbiose Agro) + TX; Trichoderma harzianum + TX; Trichoderma harzianum rifai T39 (e.g. Trichodex^{®} from Makhteshim, US) + TX; Trichoderma harzianum, strain ITEM 908 (e.g. Trianum-P from Koppert) + TX; Trichoderma harzianum, strain TH35 (e.g. Root-Pro by Mycontrol) + TX; Trichoderma harzianum, strain DB 103 (available as T-GRO^{®} 7456 by Dagutat Biolab) + TX; Trichoderma polysporum, strain IMI 206039 (e.g. Binab TF WP by BINAB Bio-Innovation AB, Sweden) + TX; Trichoderma stromaticum, having Accession No. Ts3550 (e.g. Tricovab by CEPLAC, Brazil) + TX; Trichoderma virens (also known as Gliocladium virens), in particular strain GL-21 (e.g. SoilGard by Certis, US) + TX; Trichoderma virens strain G-41, formerly known as Gliocladium virens (Accession No. ATCC 20906) (e.g., ROOTSHIELD^{®} PLUS WP and TURFSHIELD^{®} PLUS WP from BioWorks, US) + TX; Trichoderma viride, strain TV1 (e.g. Trianum-P by Koppert) + TX; Trichoderma viride, in particular strain B35 (Pietr et al., 1993, Zesz. Nauk. A R w Szczecinie 161: 125-137) + TX; mixtures of Trichoderma asperellum strain ICC 012 (also known as Trichoderma harzianum ICC012), having Accession No. CABI CC IMI 392716 and Trichoderma gamsii (formerly T. viride) strain ICC 080, having Accession No. IMI 392151 (e.g., BIO-TAM^{™} from Isagro USA, Inc. and BIODERMA^{®} by Agrobiosol de Mexico, S.A. de C.V.) + TX; Ulocladium oudemansii strain U3, having Accession No. NM 99/06216 (e.g., BOTRY-ZEN^{®} by Botry-Zen Ltd, New Zealand and BOTRYSTOP^{®} from BioWorks, Inc.) + TX; Verticillium albo-atrum (formerly V. dahliae), strain WCS850 having Accession No. WCS850, deposited at the Central Bureau for Fungi Cultures (e.g., DUTCH TRIG^{®} by Tree Care Innovations) + TX; Verticillium chlamydosporium + TX;
(3) **biological control agents having an effect for improving plant growth and/or plant health** selected from the group of:
   (3.1) bacteria, examples of which are *Azospirillum brasilense* (e.g., VIGOR^{®} from KALO, Inc.) + TX; *Azospirillum lipoferum* (e.g., VERTEX-IF^{™} from TerraMax, Inc.) + TX; *Azorhizobium caulinodans,* in particular strain ZB-SK-5 + TX; *Azotobacter chroococcum,* in particular strain H23 + TX; *Azotobacter vinelandii*, in particular strain ATCC 12837 + TX; a mixture of *Azotobacter vinelandii* and *Clostridium pasteurianum* (available as INVIGORATE^{®} from Agrinos) + TX; *Bacillus amyloliquefaciens* pm414 (LOLI-PEPTA^{®} from Biofilm Crop Protection) + TX; *Bacillus amyloliquefaciens* SB3281 (ATCC # PTA-7542, WO 2017/205258) + TX; *Bacillus amyloliquefaciens* TJ1000 (available as QUIKROOTS^{®} from Novozymes) + TX; *Bacillus amyloliquefaciens,* in particular strain IN937a + TX; *Bacillus amyloliquefaciens,* in particular strain FZB42 (e.g. RHIZOVITAL^{®} from ABiTEP, DE) + TX; *Bacillus amyloliquefaciens* BS27 (Accession No. NRRL B-5015) + TX; *Bacillus cereus* family member EE128 (NRRL No. B-50917) + TX; *Bacillus cereus* family member EE349 (NRRL No. B-50928) + TX; *Bacillus cereus,* in particular strain BP01 (ATCC 55675, e.g. MEPICHLOR^{®} from Arysta Lifescience, US) + TX; *Bacillus firmus,* in particular strain CNMC 1-1582 (e.g. VOTIVO^{®} from BASF SE) + TX; *Bacillus mycoides* BT155 (NRRL No. B-50921) + TX; *Bacillus mycoides* EE118 (NRRL No. B-50918) + TX; *Bacillus mycoides* EE141 (NRRL No. B-50916) + TX; *Bacillus mycoides* BT46-3 (NRRL No. B-50922) + TX; *Bacillus pumilus,* in particular strain QST2808 (having Accession No. NRRL No. B-30087) + TX; *Bacillus pumilus,* in particular strain GB34 (e.g. YIELD SHIELD^{®} from Bayer Crop Science, DE) + TX; *Bacillus siamensis,* in particular strain KCTC 13613T + TX; *Bacillus subtilis,* in particular strain QST713/AQ713 (having NRRL Accession No. B-21661 and described in U.S. Patent No. 6,060,051, available as SERENADE^{®} OPTI or SERENADE^{®} ASO from Bayer CropScience LP, US) + TX; *Bacillus subtilis,* in particular strain AQ30002 (having Accession Nos. NRRL B-50421 and described in U.S. Patent Application No. 13/330,576) + TX; *Bacillus subtilis,* in particular strain AQ30004 (and NRRL B-50455 and described in U.S. Patent Application No. 13/330,576) + TX; *Bacillus subtilis* strain BU1814, (available as TEQUALIS^{®} from BASF SE), *Bacillus* subtilis rm303 (RHIZOMAX^{®} from Biofilm Crop Protection) + TX; *Bacillus thuringiensis* BT013A (NRRL No. B-50924) also known as *Bacillus thuringiensis* 4Q7 + TX; a mixture of *Bacillus licheniformis* FMCH001 and *Bacillus subtilis* FMCH002 (available as QUARTZO^{®} (WG), PRESENCE^{®} (WP) from FMC Corporation) + TX; *Bacillus subtilis,* in particular strain MBI 600 (e.g. SUBTILEX^{®} from BASF SE) + TX; *Bacillus tequilensis,* in particular strain NII-0943 + TX; *Bradyrhizobium japonicum* (e.g. OPTIMIZE^{®} from Novozymes) + TX; *Delftia acidovorans,* in particular strain RAY209 (e.g. BIOBOOST^{®} from Brett Young Seeds) + TX; *Mesorhizobium cicer* (e.g., NODULATOR from BASF SE) + TX; *Lactobacillus* sp. (e.g. LACTOPLANT^{®} from LactoPAFI) + TX; *Rhizobium leguminosarium biovar viciae* (e.g., NODULATOR from BASF SE) + TX; Pseudomonas proradix (e.g. PRORADIX^{®} from Sourcon Padena) + TX; Pseudomonas aeruginosa, in particular strain PN1 + TX; Rhizobium leguminosarum, in particular bv. viceae strain Z25 (Accession No. CECT 4585) + TX; Paenibacillus polymyxa, in particular strain AC-1 (e.g. TOPSEED^{®} from Green Biotech Company Ltd.) + TX; Serratia marcescens, in particular strain SRM (Accession No. MTCC 8708) + TX; Sinorhizobium meliloti strain NRG-185-1 (NITRAGIN^{®} GOLD from Bayer CropScience) + TX; Thiobacillus sp. (e.g. CROPAID^{®} from Cropaid Ltd UK) + TX; and
   (3.2) fungi, examples of which are *Purpureocillium lilacinum* (previously known as *Paecilomyces lilacinus*) strain 251 (AGAL 89/030550, e.g. BioAct from Bayer CropScience Biologics GmbH) + TX; *Penicillium bilaii,* strain ATCC 22348 (e.g. JumpStart^{®} from Acceleron BioAg), *Talaromyces flavus,* strain V117b + TX; Trichoderma atroviride strain CNCM 1-1237 (e.g. Esquive^{®} WP from Agrauxine, FR), Trichoderma viride, e.g. strain B35 (Pietr et al., 1993, Zesz. Nauk. A R w Szczecinie 161: 125-137) + TX; Trichoderma atroviride strain LC52 (also known as Trichoderma atroviride strain LU132, e.g. Sentinel from Agrimm Technologies Limited) + TX; Trichoderma atroviride strain SC1 described in International Application No. PCT/IT2008/000196) + TX;Trichoderma asperellum strain kd (e.g. T-Gro from Andermatt Biocontrol) + TX; Trichoderma asperellum strain Eco-T (Plant Health Products, ZA), Trichoderma harzianum strain T-22 (e.g. Trianum-P from Andermatt Biocontrol or Koppert) + TX; Myrothecium verrucaria strain AARC-0255 (e.g. DiTera^{™} from Valent Biosciences) + TX; Penicillium bilaii strain ATCC ATCC20851 + TX; Pythium oligandrum strain M1 (ATCC 38472, e.g. Polyversum from Bioprepraty, CZ) + TX; Trichoderma virens strain GL-21 (e.g. SoilGard^{®} from Certis, USA) + TX; Verticillium albo-atrum (formerly V. dahliae) strain WCS850 (CBS 276.92, e.g. Dutch Trig from Tree Care Innovations) + TX; Trichoderma atroviride, in particular strain no. V08/002387, strain no. NMI No. V08/002388, strain no. NMI No. V08/002389, strain no. NMI No. V08/002390 + TX; Trichoderma harzianum strain ITEM 908, Trichoderma harzianum, strain TSTh20 + TX; Trichoderma harzianum strain 1295-22 + TX; Pythium oligandrum strain DV74 + TX; Rhizopogon amylopogon (e.g. comprised in Myco-Sol from Helena Chemical Company) + TX; Rhizopogon fulvigleba (e.g. comprised in Myco-Sol from Helena Chemical Company) + TX;Trichoderma virens strain GI-3 + TX;
(4) **insecticidally active biological control agents** selected from
   (4.1) bacteria, examples of which are *Agrobacterium radiobacter* strain K84 (Galltrol from AgBiochem Inc.) + TX; *Bacillus amyloliquefaciens,* in particular strain PTS-4838 (e.g. AVEO from Valent Biosciences, US) + TX; *Bacillus firmus,* in particular strain CNMC 1-1582 (e.g. VOTIVO^{®} from BASF SE) + TX; *Bacillus mycoides,* isolate J. (e.g. BmJ from Certis USA LLC, a subsidiary of Mitsui & Co.) + TX; *Bacillus sphaericus,* in particular Serotype H5a5b strain 2362 (strain ABTS-1743) (e.g. VECTOLEX^{®} from Valent BioSciences, US) + TX; *Bacillus thuringiensis* subsp. aizawai, in particular strain ABTS-1857 (SD-1372, e.g. XENTARI^{®} from Valent BioSciences) + TX; *Bacillus thuringiensis* subsp. aizawai, in particular serotype H-7 (e.g. FLORBAC^{®} WG from Valent BioSciences, US) + TX; *Bacillus thuringiensis israelensis* strain BMP 144 (e.g. AQUABAC^{®} by Becker Microbial Products IL) + TX; *Bacillus thuringiensis* subsp. *israelensis* (serotype H-14) strain AM65-52 (Accession No. ATCC 1276) (e.g. VECTOBAC^{®} by Valent BioSciences, US) + TX; *Bacillus thuringiensis* subsp. *aizawai* strain GC-91 + TX; Bacillus thuringiensis var. Colmeri (e.g. TIANBAOBTC by Changzhou Jianghai Chemical Factory) + TX; Bacillus thuringiensis var. japonensis strain Buibui + TX; Bacillus thuringiensis subsp. kurstaki strain BMP 123 from Becker Microbial Products, IL + TX; Bacillus thuringiensis subsp. kurstaki strain BMP 123 by Becker Microbial Products, IL, e.g. BARITONE from Bayer CropScience + TX; Bacillus thuringiensis subsp. kurstaki strain HD-1 (e.g. DIPEL^{®} ES from Valent BioSciences, US) + TX; Bacillus thuringiensis var. kurstaki strain EVB-113-19 (e.g., BIOPROTEC^{®} from AEF Global) + TX; Bacillus thuringiensis subsp. kurstaki strain ABTS 351 + TX; Bacillus thuringiensis subsp. kurstaki strain PB 54 + TX; Bacillus thuringiensis subsp. kurstaki strain SA 11, (JAVELIN from Certis, US) + TX; Bacillus thuringiensis subsp. kurstaki strain SA 12 (THURICIDE from Certis, US) + TX; Bacillus thuringiensis subsp. kurstaki strain EG 2348 (LEPINOX from Certis, US) + TX; Bacillus thuringiensis subsp. kurstaki strain EG 7841 (CRYMAX from Certis, US) + TX; Bacillus thuringiensis subsp. tenebrionis strain NB 176 (SD-5428, e.g. NOVODOR^{®} FC from BioFa DE) + TX; Brevibacillus laterosporus (LATERAL from Ecolibrium Biologicals) + TX; Burkholderia spp., in particular Burkholderia rinojensis strain A396 (also known as Burkholderia rinojensis strain MBI 305) (Accession No. NRRL B-50319 + TX; WO 2011/106491 and WO 2013/032693 + TX; e.g. MBI206 TGAI and ZELTO^{®} from Marrone Bio Innovations) + TX; Chromobacterium subtsugae, in particular strain PRAA4-1T (MBI-203 + TX; e.g. GRANDEVO^{®} from Marrone Bio Innovations) + TX; Lecanicillium muscarium Ve6 (MYCOTAL from Koppert) + TX; Paenibacillus popilliae (formerly Bacillus popilliae + TX; e.g. MILKY SPORE POWDER^{™} and MILKY SPORE GRANULAR^{™} from St. Gabriel Laboratories) + TX; Pasteuria nishizawae strain Pn1 (CLARIVA from Syngenta/ChemChina) + TX;Serratia entomophila (e.g. INVADE^{®} by Wrightson Seeds) + TX; Serratia marcescens, in particular strain SRM (Accession No. MTCC 8708) + TX;Trichoderma asperellum (TRICHODERMAX from Novozymes) + TX; Wolbachia pipientis ZAP strain (e.g., ZAP MALES^{®} from MosquitoMate) + TX; and
   (4.2) fungi, examples of which are *Beauveria bassiana* strain ATCC 74040 (e.g. NATURALIS^{®} from Intrachem Bio Italia) + TX; *Beauveria bassiana* strain GHA (Accession No. ATCC74250, e.g. BOTANIGUARD^{®} ES and MYCONTROL-O^{®} from Laverlam International Corporation) + TX; *Beauveria bassiana* strain ATP02 (Accession No. DSM 24665) + TX;*Isaria fumosorosea* (previously known as *Paecilomyces fumosoroseus*) strain Apopka 97) PREFERAL from SePRO + TX; *Metarhizium anisopliae* 3213-1 (deposited under NRRL accession number 67074) (WO 2017/066094 + TX; Pioneer Hi-Bred International) + TX; *Metarhizium robertsii* 15013-1 (deposited under NRRL accession number 67073) + TX; *Metarhizium robertsii* 23013-3 (deposited under NRRL accession number 67075) + TX; *Paecilomyces lilacinus* strain 251 (MELOCON from Certis, US) + TX; *Zoophtora radicans* + TX;
(5) Viruses selected from the group consisting of *Adoxophyes orana* (summer fruit tortrix) granulosis virus (GV) + TX; *Cydia pomonella* (codling moth) granulosis virus (GV) + TX; Helicoverpa armigera (cotton bollworm) nuclear polyhedrosis virus (NPV) + TX; *Spodoptera exigua* (beet armyworm) mNPV + TX; *Spodoptera frugiperda* (fall armyworm) mNPV + TX; *Spodoptera littoralis* (African cotton leafworm) NPV + TX;
(6) **Bacteria and fungi which can be added as 'inoculant' to plants or plant parts or plant organs and which, by virtue of their particular properties, promote plant growth and plant health** selected from *Agrobacterium spp.* + *TX;* Azorhizobium caulinodans + TX; Azospirillum spp. + TX; Azotobacter spp. + TX; Bradyrhizobium spp. + TX; Burkholderia spp., in particular Burkholderia cepacia (formerly known as Pseudomonas cepacia) + TX; Gigaspora spp., or Gigaspora monosporum + TX; Glomus spp. + TX; Laccaria spp. + TX; LactoBacillus buchneri + TX; Paraglomus spp. + TX; Pisolithus tinctorus + TX; Pseudomonas spp. + TX; Rhizobium spp., in particular Rhizobium trifolii + TX; Rhizopogon spp. + TX; Scleroderma spp. + TX; Suillus spp. + TX; Streptomyces spp. + TX;
(7) **Plant extracts and products formed by microorganisms including proteins and secondary metabolites which can be used as biological control agents,** selected from *Allium sativum* (NEMGUARD from Eco-Spray + TX; BRALIC from ADAMA) + TX; Armour-Zen + TX; *Artemisia absinthium* + TX; Azadirachtin (e.g. AZATIN XL from Certis, US) + TX; Biokeeper WP + TX; Brassicaceae extract, in particular oilseed rape powder or mustard powder + TX; Cassia nigricans + TX; Celastrus angulatus + TX; Chenopodium anthelminticum + TX; Chitin + TX; Dryopteris filix-mas + TX; Equisetum arvense + TX; Fortune Aza + TX; Fungastop + TX; Heads Up (Chenopodium quinoa saponin extract) + TX; PROBLAD (naturally occurring Blad polypeptide from Lupin seeds), Certis EU + TX; FRACTURE (naturally occurring Blad polypeptide from Lupin seeds), FMC + TX; Pyrethrum/Pyrethrins + TX; Quassia amara + TX; Quercus + TX; Quillaja extract (QL AGRI 35 from BASF) + TX; Reynoutria sachalinensis extract (REGALLIA / REGALIA MAXX from Marrone Bio) + TX; "Requiem ^{™} Insecticide" + TX; Rotenone + TX; ryania/ryanodine + TX; Symphytum officinale + TX; Tanacetum vulgare + TX; Thymol + TX; Thymol mixed with Geraniol (CEDROZ from Eden Research) + TX; Thymol mixed with Geraniol and Eugenol (MEVALONE from Eden Research) + TX; Triact 70 + TX; TriCon + TX; Tropaeulum majus + TX; Melaleuca alternifolia extract (TIMOREX GOLD from STK) + TX; Urtica dioica + TX; Veratrin + TX; and Viscum album + TX; and
   a safener, such as benoxacor + TX, cloquintocet (including cloquintocet-mexyl) + TX, cyprosulfamide + TX, dichlormid + TX, fenchlorazole (including fenchlorazole-ethyl) + TX, fenclorim + TX, fluxofenim + TX, furilazole + TX, isoxadifen (including isoxadifen-ethyl) + TX, mefenpyr (including mefenpyr-diethyl) + TX, metcamifen + TX and oxabetrinil + TX.

The references in brackets behind the active ingredients, e.g. *[*3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/ChemicalAbstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula I selected from the compounds defined in the Tables A-1 to A-7, B-1- to B-7, C-1 to C-7 and P with active ingredients described above comprises a compound selected from one compound defined in the Tables A-1 to A-7, B-1- to B-7, C-1 to C-7 and P and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 to 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The compounds and mixtures as described above can be used in a method for controlling pests, which comprises applying a composition comprising a compound or mixture respectively as described above to the pests or their environment, with the exception of a method for treatment of the human or animal body by surgery or therapy and diagnostic methods practised on the human or animal body.

The mixtures comprising a compound of formula I selected from the compounds defined in the Tables A-1 to A-7, B-1- to B-7, C-1 to C-7 and P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha, especially 10 to 200 g/ ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of formula I of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula I. The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula I. Further, it is hereby made available, a composition comprising a plant propagation material treated with a compound of formula I.

Seed treatment comprises all suitable seed treatment techniques known in the art, such as seed dressing, seed coating, seed dusting, seed soaking and seed pelleting. The seed treatment application of the compound formula I can be carried out by any known methods, such as spraying or by dusting the seeds before sowing or during the sowing/planting of the seeds.

The compounds of the invention can be distinguished from other similar compounds by virtue of greater efficacy at low application rates and/or different pest control, which can be verified by the person skilled in the art using the experimental procedures, using lower concentrations if necessary, for example 10 ppm, 5 ppm, 2 ppm, 1 ppm or 0.2 ppm; or lower application rates, such as 300, 200 or 100, mg of Al per m². The greater efficacy can be observed by an increased safety profile (against non-target organisms above and below ground (such as fish, birds and bees), improved physicochemical properties, or increased biodegradability).

In each aspect and embodiment of the invention, "consisting essentially" and inflections thereof are a preferred embodiment of "comprising" and its inflections, and "consisting of" and inflections thereof are a preferred embodiment of "consisting essentially of" and its inflections.

The disclosure in the present application makes available each and every combination of embodiments disclosed herein.

It should be noted that the disclosure herein in respect of a compound of formula I applies equally in respect of a compound of each of formulae Ia, Ib, Ic and Id, and Tables A-1 to A-7, B-1- to B-7, C-1 to C-7 and P.

The compounds of the invention can be distinguished from other similar compounds by virtue of greater efficacy at low application rates and/or different pest control, which can be verified by the person skilled in the art using the experimental procedures, using lower concentrations if necessary, for example 10 ppm, 5 ppm, 2 ppm, 1 ppm or 0.2 ppm; or lower application rates, such as 300, 200 or 100, mg of Al per m². The greater efficacy can be observed by an increased safety profile (against non-target organisms above and below ground (such as fish, birds and bees), improved physicochemical properties, or increased biodegradability).

### Biological Examples:

The Examples which follow serve to illustrate the invention. Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 24 ppm, 12.5 ppm, 6 ppm, 3 ppm, 1.5 ppm, 0.8 ppm or 0.2 ppm.

### Resistant Plutella xylostella (Diamond back moth) larvicide L3, feeding/contact

Chinese cabbage plants were sprayed with diluted test solutions in an application chamber. Cut off leaves were placed into petri dishes with wetted filter paper and infested 1 day after application with 10 L3 multi- resistant *Plutella xylostella* larvae having the G4946E resistance mutation.

Samples were assessed 4 days after infestation for mortality and growth regulation.

### Origin:

Resistant strain: ThaiR a field-collected diamide resistant strain was collected from Bang Bua Thongin in Thailand in 2012. The strain is reared on cabbage plants (*Brassica aleracea*) and selected approximately every two weeks with chlorantraniliprole.

The following compounds, according to the present invention, gave at least 80% control of the resistant strain of *Plutella xylostella* at 50ppm or below:
P.1, P.2

12 out of 16 test series of chlorantraniliprole did not control the resistant *Plutella* strain at 80% at 50 ppm or below; whereas the chlorantraniliprole was controlling the sensitive *Plutella* strain at 80% at 0.25 ppm on average.

### In Vitro assay method description

Human embryonic kidney cells expressing the *Plutella xylostella* (Diamond back moth) ryanodine receptor, containing the G4946E resistance mutation, are loaded with Fluo-8 No Wash (NW) calcium-sensitive dye which responds by fluorescence to a change in intracellular calcium (e.g. stimulated by activation of ryanodine receptor). Test compounds are added in 10 rates to a 384-well plate containing dye-loaded cells and the fluorescent signal is measured using the Hamamatsu FDSS (Functional Drug Screening system). Dose-response curves are plotted to estimate the EC₅₀. EC₅₀ are normalized against an in-assay reference standard (cyantraniliprole) to address inter-assay variability. The ratio for a given compound is then obtained by the following formula:
R_{EC50} = EC₅₀ (compound)/ EC₅₀ (cyantraniliprole). Compounds for which the ratio R_{EC50} is inferior or equal to 1 are equally or more active than cyantraniliprole.

The following compounds, according to the present invention, obtained a ratio R_{EC50} ≤ 1:
P.1, P.2, P.4

**Table below provides the R_{EC50} for the compounds**

| Compound of the invention | | Comparative compound | |
|---|---|---|---|
| | R_{EC50} | | R_{EC50} |
| **P.2** | 0.25 | 4,6-dichloro-3-[[2-(3-chloro-2-pyridyl)-5-(2,2,2-trifluoroethoxy)pyrazole-3-carbonyl]amino]-N-methyl-pyridine-2-carboxamide | 2.66 |

## Claims

1. A method for combating and controlling diamide-resistant insects to
(i) reduce damage on a plant, which comprises applying to the insect, to a locus of the insect, or to a plant susceptible to attack by the insect, an effective amount of a compound of formula I; or
(ii) protect plant propagation material, which comprises treating the propagation material or the site, where the propagation material is planted, with an effective amount of a compound of formula I;
wherein the compound of formula I is 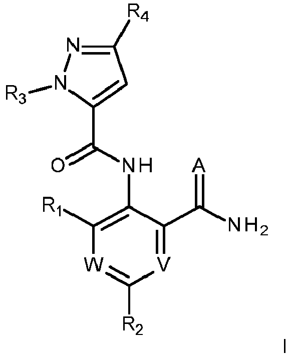 wherein
A is O or S;
V and W are independently CH or N, with the proviso that V and W cannot both be CH;
R₁ is hydrogen, halogen, C₁-C₆alkyl, C₁-C₆haloalkyl, or C₃-C₆cycloalkyl;
R₂ is hydrogen, halogen or cyano;
R₃ is phenyl, or a 6-membered heteroaromatic ring, each of which is unsubstituted or substituted with one to three substituents independently selected from R₆;
R₄ is hydrogen, halogen, C₁-C₆alkyl, C₁-C₆haloalkyl, C₃-C₆cycloalkyl, C₃-C₆halocycloalkyl, C₁-C₆alkoxy, C₁-C₆haloalkoxy, or CH₂-Y, where Y is C₃-C₆cycloalkyl, C₃-C₆halocycloalkyl, benzyloxy, halobenzyloxy, 5- or 6-membered heteroaromatic ring, which is unsubstituted or substituted with one to three groups independently selected from R₇, or a 9- or 10-membered heteroaromatic bicyclic system, which is unsubstituted or substituted with one to three groups independently selected from R₇; R₆ is independently selected from halogen, cyano, C₁-C₃alkyl, C₁-C₃haloalkyl, C₁-C₃alkoxy, and C₁-C₃haloalkoxy;
R₇ is independently selected from halogen, cyano, C₁-C₃alkyl, C₁-C₃haloalkyl, C₃-C₆cycloalkyl, phenyl, and phenyl substituted with one to three independently selected substituents from halogen, cyano, C₁-C₃alkyl, and C₁-C₃haloalkyl;
or an agronomically acceptable salt, isomer, enantiomer, tautomer and/or N-oxide of the compound of formula I.

2. The method according to claim 1, wherein the diamide-resistant insect is from the order Lepidoptera.

3. The method according to claim 1 or claim 2, wherein the diamide-resistant insect is resistant to least one compound selected from chlorantraniliprole, cyantraniliprole, cyclantraniliprole, fluchlordiniliprole, tetrachlorantraniliprole, tetraniliprole, flubendiamide and cyhalodiamide.

4. The method according to any one of claims 1 to 3, wherein compound of formula I is represented by formula Ib, Ic, or Id 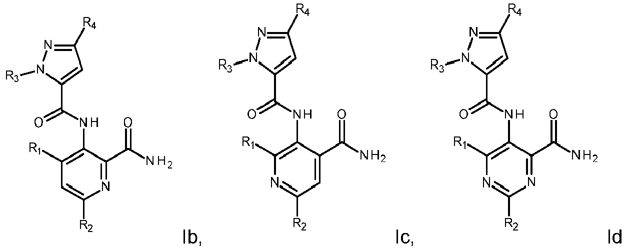 where R₁, R₂, R₃ and R₄ are as defined in claim 1.

5. The method according to any one of claims 1 to 4, wherein the diamide-resistant insect is Plutella xylostella (Troczka et al. 2012; Steinbach et al. 2015; Guo et al. 2014), Tuta absoluta (Roditakis et al. 2017; Zimmer et al. 2019), Spodoptera frugiperda (Bolzan et al. 2019) Spodoptera exigua (Zuo et al. 2020, 2017), or Chilo suppressalis (Yao et al. 2017; Yang et al. 2017).

6. The method according to any one of claims 1 to 5, wherein the diamide-resistant insect is in a defined area /field of plants where the ratio of diamide-resistant insects to their corresponding sensitive strains is greater than 1:20 (based on number of insects).

7. The method according to any one of claims 1 to 6, wherein the compound of formula I controls the diamide-resistant insect better compared to the secondary amide analog of the compound of formula I.

8. A compound of formula I as as defined in claim 1 wherein A, V, W, R₁, R₂, R₃, and R₄ as defined in claim 1, and when
• R₃ is 3-chloro-2-pyridyl, V is N, W is CH, R₄ is bromo, chloro or CF₃, and R₁ is chloro, then R₂ is hydrogen, fluoro, bromo, iodo or cyano;
• R₃ is 3-chloro-2-pyridyl, V is N, W is CH, R₄ is bromo and R₁ is bromo, then R₂ is hydrogen, fluoro, chloro, iodo or cyano; and
• R₃ is 3-chloro-2-pyridyl, V is CH, W is N, R₄ is bromo and R₁ is chloro, then R₂ is hydrogen, fluoro, bromo, iodo or cyano.

9. The compound according to claim 8, wherein R₁ is methyl, chlorine or bromine; R₂ is bromine, chlorine or cyano; R₃ is 3-chloro-2-pyridyl or 3,5-dichloro-2-pyridyl; and R₄ is CH₂-Y (Y is selected from Ya1 to Ya6).

10. A composition comprising a compound of formula I as defined in either claim 8 or 9, one or more auxiliaries and diluent, and optionally one or more other active ingredient.

11. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula I as defined in either claim 8 or 9, or a composition as defined in claim 10.

12. A method for the protection of plant propagation material from the attack by insects, acarines, nematodes or molluscs, which comprises treating the propagation material or the site, where the propagation material is planted, with an effective amount of a compound of formula I as defined in either claim 8 or 9, or a composition as defined in claim 10.

13. A plant propagation material, such as a seed, comprising, or treated with or adhered thereto, a compound of formula I as defined in either claim 8 or 9, or a composition as defined in claim 10.

14. A compound of formula IV 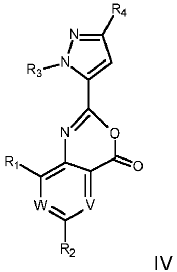 wherein W, V, are as defined in claim 1, R₁ and R₂ are each chlorine, R₃ is 3-chloro-pyrid-2-yl ring or 3,5-dichloro-pyrid-2-yl ring, and R₄ is C₁-C₃alkoxy, C₁-C₃haloalkoxy, or CH₂-Y (where Y is Ya1 to Ya17).
